(19) **Europäisches Patentamt / European Patent Office / Office européen des brevets**

(11) **EP 4 428 635 B1**

(12) **EUROPEAN PATENT SPECIFICATION**

(45) Date of publication and mention
of the grant of the patent:
**19.11.2025 Bulletin 2025/47**

(21) Application number: **22910441.9**

(22) Date of filing: **05.08.2022**

(51) International Patent Classification (IPC):
**G05B 13/04** (2006.01) **G05B 13/02** (2006.01)

(52) Cooperative Patent Classification (CPC):
**G05B 13/048; B01J 19/0033; C10G 9/00;
C10G 11/187; C10G 47/36; G05B 13/0265**

(86) International application number:
**PCT/JP2022/030118**

(87) International publication number:
**WO 2023/119711 (29.06.2023 Gazette 2023/26)**

(54) **PROGRAM, INFORMATION PROCESSING DEVICE, AND METHOD**

PROGRAMM, INFORMATIONSVERARBEITUNGSVORRICHTUNG UND VERFAHREN

PROGRAMME, DISPOSITIF DE TRAITEMENT D'INFORMATIONS, ET PROCÉDÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.12.2021 PCT/JP2021/047814**

(43) Date of publication of application:
**11.09.2024 Bulletin 2024/37**

(73) Proprietor: **Chiyoda Corporation
Yokohama 220-8765 (JP)**

(72) Inventors:
• **SATO, Hideki
Yokohama-shi, Kanagawa 220-8765 (JP)**

• **KAWAI, Eiji
Yokohama-shi, Kanagawa 220-8765 (JP)**

(74) Representative: **Gulde & Partner
Patent- und Rechtsanwaltskanzlei mbB
Berliner Freiheit 2
10785 Berlin (DE)**

(56) References cited:
WO-A1-2018/216746      WO-A1-2019/159280
WO-A1-2021/157667      WO-A1-2021/245910
CN-B- 101 526 814      JP-A- 2003 233 402
JP-A- 2004 008 908      JP-A- 2018 092 511
JP-A- 2020 149 677      JP-A- H0 784 606
US-A1- 2006 047 347

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

# EP 4 428 635 B1

**Description**

**Technical Field**

The present invention relates to an information processing device and a method a program.

**Background Art**

In order to provide a method for predicting the operating conditions of a petroleum complex that easily predicts an operating condition of a device that give an optimal value of an objective function in linear programming, there is a technology that simulates a relationship between an operating condition and a product yield.

JP 2002-329187 A provides a petrochemical complex operating condition estimating method capable of quickly, stably and simply estimating the operating condition of a facility for giving the optimum value of an objective function in linear programming.

US 2006/047347 A1 relates to techniques for enhanced control of processes, such as those utilized for air pollution control.

WO 2021/157667 A1 aims to improve prediction accuracy for a process including reactions in a chemical plant.

**Summary of Invention**

**Technical Problem**

With conventional technology, a reaction state of heavy oil, such as an index value such as parameter related to a reaction speed is determined by a simulation, and there was a problem that it is not possible to predict the reaction state more accurately.

The reaction states of various chemical reactions in the process of manufacturing not only for a heavy oil but also products such as chemicals (e.g. industrial chemicals, chemical fertilisers, paper, pulp, rubber, synthetic fibres, synthetic resins, petroleum products, pharmaceuticals, dyes, detergents, cosmetics and biotechnology products are determined through experimental data and observations of actually operating reaction device, and there was a problem that it is not possible to predict the reaction state more accurately.

An object of the present disclosure is to provide a technology that can accurately predict a reaction state and control the reaction.

**Solution to Problem**

An information processing device including a processor according to appended claim 1, a method for a computer including a processor according to appended claim 6 and a corresponding program according to appended claim 7 are provided.

An information processing device according to the present disclosure is an information processing device including a processor. The information processing device executes a step of accepting input as learning data of values indicating an oil quality of a heavy oil that is subject to a heavy oil decomposition, an operational parameter related to a heavy oil decomposition device that performs a heavy oil decomposition, and a reaction state when the heavy oil decomposition is performed in the heavy oil decomposition device using the operating parameter, a step of learning, using the learning data, a first model that outputs the reaction state of the heavy oil decomposition in response to inputting the oil quality of the heavy oil and the operating parameter, and a step of storing the learned first model in a storage unit.

According to a program according to the present disclosure, it is possible to accurately predict the reaction state of the heavy oil decomposition.

**Brief Description of Drawings**

FIG. 1 is a block diagram showing a configuration of an information processing system 1.
FIG. 2 is a block diagram showing a configuration of an information processing device 10.
FIG. 3 is a diagram showing a configuration example of a heavy oil decomposition device 20.
FIG. 4 is a block diagram showing a functional configuration of the information processing device 10.
FIG. 5 is a flowchart showing an example of a flow of learning processing performed by the information processing device 10.

FIG. 6 is a flowchart showing an example of a flow of prediction processing performed by the information processing device 10.

FIG. 7 is a flowchart showing an example of a flow of relearning processing performed by the information processing device 10.

FIG. 8 is a flowchart showing an example of a flow of product yield calculation processing performed by the information processing device 10.

FIG. 9 is a flowchart showing an example of a flow of optimization processing performed by the information processing device 10.

FIG. 10 is a diagram showing a configuration example of the heavy oil decomposition device 20.

FIG. 11 is a diagram showing a configuration example of the heavy oil decomposition device 20.

FIG. 12 is a block diagram showing the configuration of an information processing system 2.

FIG. 13 is a block diagram showing a functional configuration of an information processing device 50.

FIG. 14 is a diagram showing an example of a data structure of a first DB 521.

FIG. 15 is a diagram showing an example of a data structure of a second DB 522.

FIG. 16 is a diagram showing an example of a data structure of a third DB 523.

FIG. 17 is a diagram showing an example of a quantified reaction rate-limiting correlation.

FIG. 18 is a flowchart showing an example of a flow of learning processing performed by the information processing device 50.

FIG. 19 is a flowchart showing an example of a flow of calculation processing in step S502.

FIG. 20 is a flowchart showing an example of a flow of prediction processing performed by the information processing device 50.

**Description of Embodiments**

Embodiments of the present disclosure will be described below with reference to the drawings. In the following description, the same parts are given the same reference numerals. Their names and functions are also the same. Therefore, detailed description thereof will not be repeated.

<SUMMARY OF THIS DISCLOSURE>

The technology of the present disclosure relates to a technology for predicting reaction states in chemical reactions such as heavy oil decomposition and chemical products (e.g., industrial chemicals, chemical fertilizers, paper, pulp, rubber, synthetic fibers, synthetic resins, petroleum products, pharmaceuticals, dyes, detergents, cosmetics, bioproducts), etc.

<FIRST EMBODIMENT>

The first embodiment shows a technology that predicts values indicating the reaction state when the heavy oil decomposition is performed based on an operating parameter of a heavy oil decomposition device that performs the heavy oil decomposition and an oil quality of the heavy oil.

In the present disclosure, the heavy oil is oil extracted from the bottom of an atmospheric distillation column or a vacuum distillation column when crude oil is distilled, or a crude oil equivalent thereto. In the present disclosure, an example where the heavy oil is extracted from the bottom of an atmospheric distillation column will be described. Since the decomposition reaction of the heavy oil is a complex reaction affected by various operating parameter, it was difficult to quantify, control, and optimize the reaction state in real time during actual operation. It was difficult to accurately predict the reaction state by simply performing a simulation because the reaction state changes from moment to moment in a heavy oil decomposition device in actual operation.

The technology of the present disclosure prepares a model that learned the relationship between values indicating the reaction state of the heavy oil, and, the operating parameter and the oil quality of the heavy oil. The reaction state of the heavy oil is, for example, a parameter related to the reaction speed of the oily component of the heavy oil, an index value indicating equilibrium catalytic activity, etc. The index value indicating the equilibrium catalyst activity is, for example, the value of the activity of the equilibrium catalyst obtained by analysis, the amount of metal components deposited on the catalyst, etc.

Using this model, the information processing system 1 predicts and quantifies values indicating the reaction state of the heavy oil in real time. The information processing system 1 can control the reaction by inputting the value indicating the reaction state and the operating parameter to a physical model prepared in advance to predict product yield, etc. In this way, by optimizing the decomposition reaction of the heavy oil and contributing to the reduction of OPEX such as improving product yield and reducing the amount of catalyst input, it is also possible to contribute to improving the refinery revenues.

< 1. CONFIGURATION OF INFORMATION PROCESSING SYSTEM 1>

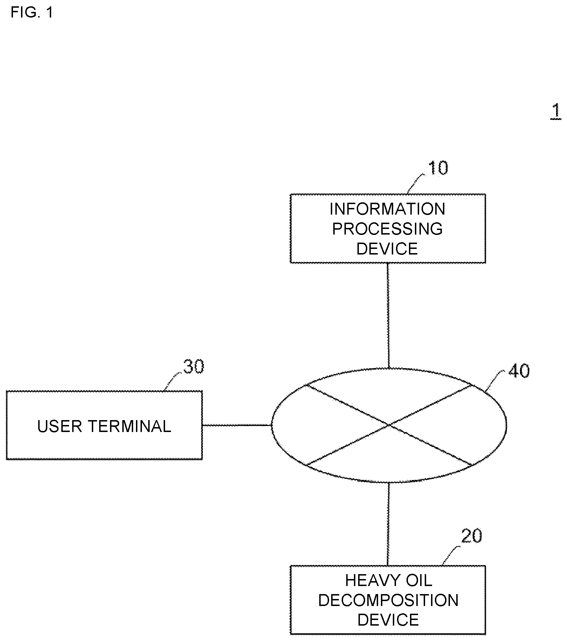

The information processing system 1 according to the present disclosure will be described using FIG. 1. The information processing system 1 according to the present disclosure includes the information processing device 10, a heavy oil decomposition device 20, a user terminal 30, and a network 40.

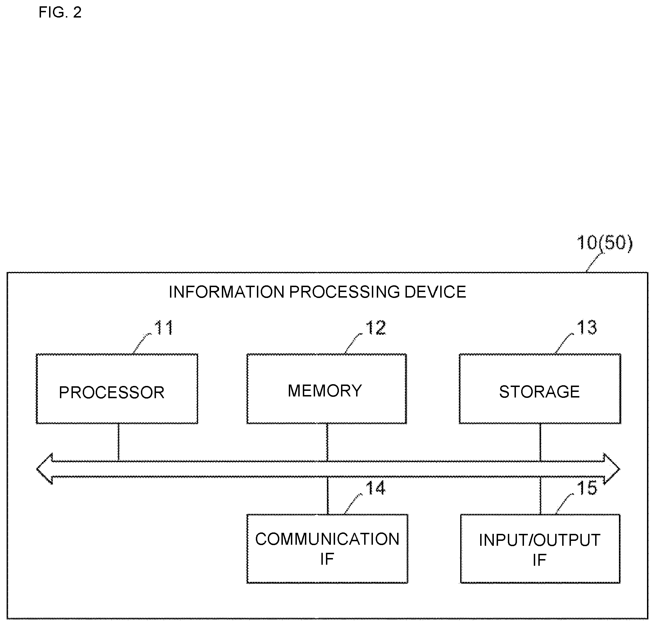

FIG. 2 is a diagram showing the configuration of the information processing device 10. The information processing device 10 is, for example, a laptop computer, a rack-mounted or tower-type computer, a smartphone, etc. Further, the information processing device 10 may be configured as one system by a plurality of information processing devices, or may be configured with redundancy. How to allocate the plurality of functions required to implement the information processing device 10 can be determined as appropriate in view of the processing capacity of each piece of hardware, the specifications required of the information processing device 10, etc.

The information processing device 10 includes a processor 11, a memory 12, a storage 13, a communication IF 14, and an input/output IF 15.

The processor 11 is hardware for executing a set of instructions written in a program, and is composed of an arithmetic unit, registers, peripheral circuits, etc.

The memory 12 is for temporarily storing programs and data processed by the programs, and is, for example, a volatile memory such as DRAM (Dynamic Random Access Memory).

The storage 13 is a storage device for storing data, and is, for example, a flash memory, an HDD (Hard Disc Drive), or an SSD (Solid State Drive).

The communication IF 14 is an interface for inputting and outputting signals so that the information processing device 10 communicates with an external device. The communication IF 14 is connected to a network 40 such as a LAN, the Internet, or wide area Ethernet by wire or wirelessly.

The input/output IF 15 functions as an interface with an input device (for example, a pointing device such as a mouse, a keyboard) for receiving input operations, and an output device (display, speaker, etc.) for presenting information.

The heavy oil decomposition device 20 is a device that decomposes heavy oil into light decomposed oil through a predetermined decomposition reaction. Examples of decomposed oil include light gas (including LPG), gasoline, and middle distillates. The heavy oil decomposition device 20 decomposes heavy oil by, for example, fluid catalytic cracking (FCC), thermal cracking, hydrocracking, etc. In the following, the FCC will be explained as the heavy oil decomposition device 20.

The heavy oil decomposition device 20 has a function of controlling a reactor 21 and a regenerator 22, and a function of transmitting and receiving predetermined information to and from the information processing device 10 through communication.

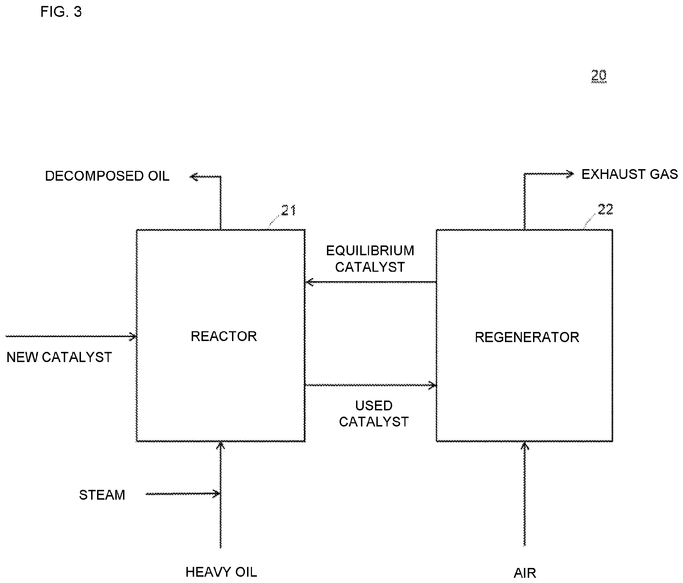

FIG. 3 is a diagram showing an example of the configuration of the heavy oil decomposition device 20. The example in FIG. 3 is a case where the heavy oil decomposition device 20 decomposes heavy oil using FCC. As shown in FIG. 3, the heavy oil decomposition device 20 includes the reactor 21 and the regenerator 22.

The reactor 21 is a device that brings heavy oil as a raw material into contact with a catalyst to cause a decomposition reaction and obtain a decomposition product. Specifically, when heavy oil, steam, and a catalyst are introduced, the reactor 21 brings the heavy oil into contact with the catalyst. Next, the reactor 21 obtains decomposed oil by decomposing the heavy oil by a decomposition reaction caused by contacting the heavy oil with the catalyst. Furthermore, the reactor 21 introduces stream into the obtained decomposed oil to remove the decomposed oil adhering to the catalyst. The reactor 21 then outputs the decomposed oil. Additionally, the reactor 21 passes the used catalyst to the regenerator 22.

The regenerator 22 regenerates the catalyst used in the reactor 21. When a catalyst is used for a heavy oil decomposition reaction, coke (carbon) adheres to the surface of the catalyst, thereby degrading the catalyst. The regenerator 22 regenerates the catalyst by burning coke attached to the surface of the catalyst at high temperature, and supplies the regenerated catalyst to the reactor 21 so as to keep the activity within the reactor 21 constant. Moreover, the regenerator 22 discharges exhaust gas generated by burning.

The user terminal 30 is a terminal operated by a user. Here, the user is, for example, a person who operates and manages the heavy oil decomposition device 20. The user terminal 30 is, for example, a smartphone, a personal computer, etc.

The information processing device 10, the heavy oil decomposition device 20, and the user terminal 30 are configured to be able to communicate with each other via a network 40.

<2. FUNCTIONAL CONFIGURATION OF INFORMATION PROCESSING DEVICE 10>

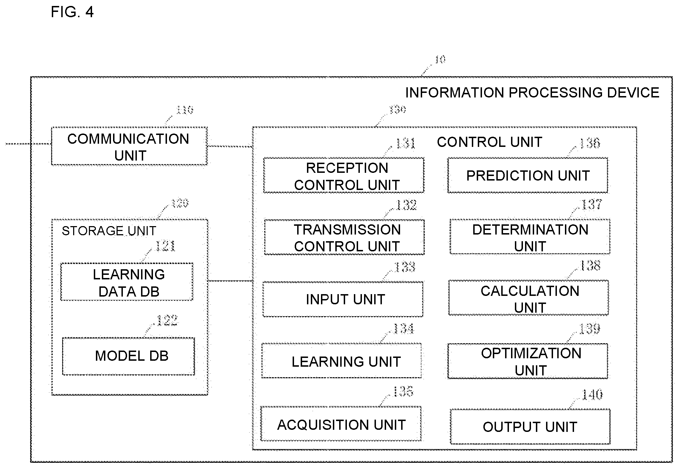

FIG. 4 is a block diagram showing the functional configuration of the information processing device 10. As shown in FIG. 2, the information processing device 10 includes a communication unit 110, a storage unit 120, and a control unit 130.

The communication unit 110 performs processing for the information processing device 10 to communicate with an external device.

The storage unit 120 stores data and programs used by the information processing device 10. The storage unit 120 stores a learning data DB 121, a model DB 122, etc.

The learning data DB 121 is a database that holds learning data used when learning processing is performed.

The learning data includes a set of the oil quality of the heavy oil that is the target of heavy oil decomposition, the operating parameter related to the heavy oil decomposition device 20 that performs heavy oil decomposition, and actual data of values indicating the reaction state when the heavy oil is decomposed in the heavy oil decomposition device 20 using the operating parameter.

The oil quality of heavy oil is information regarding the oil quality of heavy oil, such as the density, metal concentration, and nitrogen concentration, etc. of heavy oil. The operating parameter include, for example, parameter such as the flow rate of heavy oil, the amount of catalyst, the amount of steam inflow to the heavy oil decomposition device 20, the pressure, the internal temperature, the temperature of the catalyst, the ratio of the catalyst to heavy oil in the reactor 21, the pressure, internal temperature, and catalyst temperature in the regenerator 22.

For example, when the value indicating the reaction state is a parameter related to the reaction speed of heavy oil in heavy oil decomposition, the learning data adopts a first parameter related to the decomposition reaction of heavy oil among the above operating parameter as the operating parameter.

For example, parameter related to reaction speed include reaction speed constant, frequency factor, activation energy, etc. The reaction speed constant of the Arrhenius equation is expressed, for example, by the following formula. In the formula below, k is the reaction speed constant, R is the gas constant, T is the absolute temperature, E is the activation energy, and A is the frequency factor.

[Math. 1]

$$k = A \exp\left(-\frac{E}{RT}\right)$$

In the following, parameter related to reaction speed will be explained using a reaction speed constant as an example.

Further, in the learning data, for example, when the value indicating the reaction state is an index value indicating the equilibrium catalyst activity, a second parameter related to the equilibrium catalyst activity among the above-mentioned operating parameter is adopted as the operating parameter.

The model DB 122 is a database that holds various models and model parameter.

The model DB 122 holds a heavy oil reaction state prediction model (hereinafter referred to as a first model). The first model is a model that outputs the reaction state of heavy oil decomposition in response to inputting the oil quality of heavy oil and the operating parameter.

Specifically, the first model is a reaction speed constant prediction model (hereinafter referred to as a second model). The second model is a model that outputs a reaction speed constant in response to inputting the oil quality of heavy oil and the first parameter.

Further, the first model may be an equilibrium catalyst activity prediction model (hereinafter referred to as a third model). The third model is a model that outputs an index value indicating equilibrium catalyst activity in response to inputting the oil quality of heavy oil and a second parameter.

Each of the first to third models may be any model such as a machine learning model or a neural network. The first to third models may use, for example, a linear regression model to express the relationship between the oil quality of heavy oil, the operating parameter, and the reaction state of heavy oil decomposition. Furthermore, the model DB 122 may hold both the second model and the third model. The present disclosure describes an example where the model DB 122 holds both a second and a third model.

Furthermore, the model DB 122 holds models other than those described above. For example, the model DB 122 holds a physical model that outputs a product yield or an index value that contributes to the product yield by inputting the oil quality of heavy oil, the first parameter, the reaction speed constant for heavy oil decomposition determined by the second model, the second parameter, and an index value indicating equilibrium catalytic activity determined by the third model.

The control unit 130 performs the functions shown in a reception control unit 131, a transmission control unit 132, an input unit 133, a learning unit 134, an acquisition unit 135, a prediction unit 136, a determination unit 137, a calculation unit 138, an optimization unit 139, an output unit 140, etc. when the processor 11 of the information processing device 10 performs processing according to the program.

The reception control unit 131 controls a process where the information processing device 10 receives a signal from an external device according to a communication protocol.

The transmission control unit 132 controls a process where the information processing device 10 transmits a signal to an external device according to a communication protocol.

The input unit 133 accepts input of the oil quality of the heavy oil to be subjected to heavy oil decomposition, the operating parameter related to the heavy oil decomposition device 20 that performs heavy oil decomposition and a value indicating the reaction state when the heavy oil is decomposed in the heavy oil decomposition device 20 using the operating parameter, as learning data.

Specifically, the input unit 133 acquires learning data from the learning data DB 121.

The learning unit 134 learns the second model and the third model using the acquired learning data.

Specifically, the learning unit 134 learns the second model using the oil quality of the heavy oil, the first parameter, and the reaction speed constant among the learning data. For example, if the second model is a linear regression model, the learning unit 134 learns the second model using the reaction speed constant as an objective variable, the oil quality of the heavy oil and the first parameter as explanatory variables.

In the decomposition reaction of the heavy oil that has countless continuous boiling point components, the reaction control is highly difficult because not only the number of raw oil components but also multiple operating parameter interact. In actual operation, the quality of the raw oil change frequently, so it was not possible to optimize the decomposition reaction. This led to deterioration in profits, such as a decline in product yield. Particularly in decomposition reactions that use catalysts, such as FCC and hydrocracking, it is necessary to take catalyst deterioration into account, and the reaction control cannot be performed accurately, leading to an increase in OPEX due to excessive input of new catalyst.

By learning the above-mentioned second model, it is possible to learn the correlation between the reaction speed constant that changes with changes in raw oil quality and the operating parameter. Therefore, by using the learned second model, it is possible to accurately predict the reaction speed constant in actual operation.

Here, the second model learns the correlation between the first parameter and the reaction speed constant of the decomposition reaction when the heavy oil decomposition is performed using the first parameter for each oily component of the heavy oil. For example, the learning unit 134 learns the correlation between the reaction speed constant and the operating parameter that the second model outputs reaction speed constants that react with other oily components due to the heavy oil decomposition and change to each of gasoline, coke, flue gas, etc., for each of oil equivalent to vacuum residue (VR) that is the oily component of the heavy oil and oil equivalent to vacuum gas oil (VGO), etc.

By learning the second model in this way, the learning unit 134 can learn not only the number of raw oil components but also the interrelationships of multiple operating parameter, in the decomposition reaction of the heavy oil that has an infinite number of continuous boiling point components. The second model learned in this manner can accurately predict the reaction speed constant.

The learning unit 134 then stores the learned second model in the model DB 122.

The learning unit 134 also learns the third model using the oil quality of the heavy oil, the second parameter, and the index value indicating the equilibrium catalyst activity among the learning data. For example, if the third model is a linear regression model, the learning unit 134 learns the third model using the index value indicating the equilibrium catalyst activity as the objective variable, the oil quality of the heavy oil and the second parameter as the explanatory variables.

As in the above example, the catalyst circulates within the reactor 21 and regenerator 22 of the heavy oil decomposition device 20. Catalyst deterioration that occurs in the reactor 21 can be recovered to some extent in the regenerator 22. However, even in the regenerator 22, it is not possible to completely recover the deterioration of the catalyst, so in order to keep the activity of the catalyst constant, in addition to recovery in the regenerator 22, it is necessary to supply new catalyst. However, the amount of new catalyst to be introduced is determined based on past empirical rules. In actual operation, the catalyst deterioration speed varies depending on changes in the heavy oil being processed. For this reason, it was difficult to adjust the catalytic activity to the desired level.

By learning the above third model, it is possible to learn the correlation between the oil quality of the heavy oil in actual operation, the second parameter, and the index value indicating the equilibrium catalyst activity. Therefore, by using the learned third model, it is possible to accurately predict the index value indicating the equilibrium catalyst activity in actual operation. As a result, the technology of the present disclosure has the effects of statically determining the equilibrium catalyst activity, optimizing the amount of new catalyst input, and reducing the equilibrium catalyst analysis cost and labor. In particular, by using the learned third model, there is no need to consider the time required for analysis, so if the oil quality of the heavy oil and the second parameter in actual operation are available, the equilibrium catalyst activity can be obtained in real time.

The learning unit 134 then stores the learned third model in the model DB 122.

Further, the learning unit 134 can relearn first model by using the learning data, a third parameter that is an operating parameter regarding the heavy oil decomposition device 20 that is performing heavy oil decomposition, and a value that indicates the predicted reaction state. The learning unit 134 performs relearning based on, for example, the timing where the analysis results are obtained.

Specifically, the learning unit 134 acquires the learning data, a third parameter that is an operating parameter regarding the actually operating heavy oil decomposition device 20 acquired later, and a value indicating the reaction state obtained by the analysis described later, and relearns each model. For example, the learning unit 134 relearns the second model from the learning data, the first parameter of the third parameter, and the analyzed reaction speed constant. Further, for example, the learning unit 134 relearns the third model from the learning data, the second parameter of the third parameter, and the analyzed index value indicating the equilibrium catalytic activity.

In this way, the learning unit 134 relearns the second model and the third model at the timing when the analysis results are obtained, thereby obtaining index values indicating the reaction speed constant and equilibrium catalyst activity in real time with higher accuracy. For example, in order to obtain a predetermined equilibrium catalyst activity, it is necessary to adjust the amount of new catalyst to be introduced. In order to accurately determine the amount of new catalyst to be introduced, analysis results of equilibrium catalyst activity are required. This analytical result of equilibrium catalyst activity requires time for analysis after the catalyst is taken out. For this reason, it took time to reflect the analysis results in simulations used for prediction. However, since the learning unit 134 can relearn the second model and the third model at the timing when the analysis results are obtained, it is possible to obtain index values indicating the reaction speed and equilibrium catalytic activity more accurately in real time. For example, the prediction accuracy can be quickly tracked even under the influence of changes in catalysts, etc.

The acquisition unit 135 acquires a third parameter that is an operating parameter regarding a heavy oil decomposition device that is performing heavy oil decomposition, oil quality of the heavy oil, and learned second and third models.

Specifically, the acquisition unit 135 acquires the third parameter and the oil quality of the heavy oil to be introduced into the heavy oil decomposition device from the heavy oil decomposition device 20. It is noted that the acquisition unit 135 may acquire the third parameter and the oil quality of the heavy oil by receiving them from the user terminal 30. The acquisition unit 135 also acquires the learned second model and third model from the model DB 122.

Further, the acquisition unit 135 acquires the analysis result of the reaction state. Specifically, the acquisition unit 135 acquires the results of analyzing the reaction state based on actual operating parameter, decomposition products, catalysts, etc.

The prediction unit 136 predicts a value indicating the reaction state by inputting the oil quality of the heavy oil and the third parameter into the first model.

Specifically, the prediction unit 136 predicts the reaction speed constant by inputting the oil quality of the heavy oil and the first parameter of the third parameter into the second model.

Furthermore, the prediction unit 136 predicts an index value indicating the equilibrium catalyst activity by inputting the oil quality of the heavy oil and the second parameter of the third parameter into the third model.

The determination unit 137 determines the optimum amount of new catalyst to be introduced into the heavy oil decomposition device 20 based on the index value indicating the equilibrium catalyst activity.

Specifically, the determination unit 137 calculates the amount of catalyst that should be introduced to bring the equilibrium catalytic activity to a predetermined value, based on the index value indicating the equilibrium catalytic activity. Then, the determination unit 137 determines the calculated amount as the optimal amount of new catalyst.

The calculation unit 138 uses the oil quality of the heavy oil, the third parameter, and the value indicating the reaction state to calculate the product yield or an index value contributing to the product yield.

Specifically, the calculation unit 138 first obtains a reaction model from the model DB 122. Next, the calculation unit 138 calculates the product yield or an index value contributing to the product yield by using the oil quality of the heavy oil, the first parameter of the third parameter, the determined reaction speed constant for heavy oil decomposition, the second parameter of the third parameter, and the index value indicating the determined equilibrium catalyst activity, and the reaction model. The product yield is, for example, the yield of decomposed oil obtained by the heavy oil decomposition device 20. Further, the index values contributing to the product yield is operating parameter necessary for determining the product yield, etc. The index value is, for example, reaction temperature, reaction time, catalyst concentration, etc.

The optimization unit 139 uses the determined product yield or an index value contributing to the product yield, oil quality of the heavy oil, the operating parameter, and values indicating the reaction state to determine the operating parameter for optimal product yield.

Specifically, the optimization unit 139 first determines the optimum product yield by using the product yield or an index value contributing to the product yield, the oil quality of the heavy oil, the first parameter and the reaction speed constant, of the third parameter, and the second parameter and the index value indicating equilibrium catalytic activity, of the third parameter. For example, the optimization unit 139 inputs the oil quality of the heavy oil, the third parameter, the determined reaction speed, and the determined equilibrium catalyst activity into a model for searching for the optimal product yield to search for the optimal product yield.

Next, the optimization unit 139 determines optimal operating parameter by using the oil quality of the heavy oil, the first parameter and the reaction speed of the third parameter, the second parameter and an index value indicating equilibrium catalyst activity of the third parameter, and the determined optimal product yield. It is noted that the optimization unit 139 may also be configured to use the learned second model and third model to determine the optimal operating parameter.

The output unit 140 outputs the predicted reaction speed constant and index value indicating the equilibrium catalyst activity to an output device, etc. The output unit 140 may be configured to output and display the predicted reaction speed constant and the index value indicating the equilibrium catalyst activity to an external device via communication.

Furthermore, the output unit 140 outputs the determined amount of new catalyst. In response to this, when the user instructs for catalyst feeding to a catalyst feeder, etc. that is not shown in the diagram, from the user terminal 30, etc., the new catalyst is fed into the heavy oil decomposition device 20.

Further, the output unit 140 outputs the determined product yield or an index value contributing to the product yield.

Furthermore, the output unit 140 outputs the determined optimal operating parameter.

## <3. OPERATION>

Hereinafter, the processing in the information processing device 10 will be explained with reference to the drawings.

### <3.1. LEARNING PROCESSING>

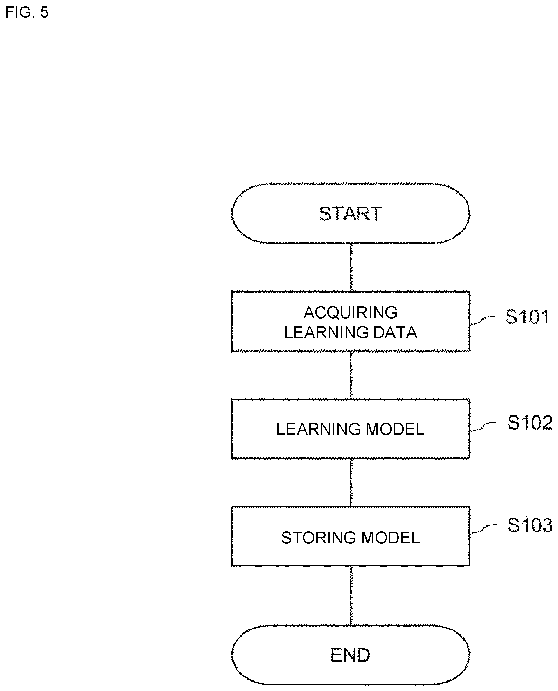

FIG. 5 is a flowchart showing an example of the flow of learning processing performed by the information processing device 10. The information processing device 10 executes the process at an arbitrary timing (for example, upon receiving a learning process start signal, etc.).

In step S101, the input unit 133 inputs oil quality of the heavy oil to be subjected to heavy oil decomposition, the operating parameter related to the heavy oil decomposition device 20 that performs heavy oil decomposition, and a value indicating a reaction state when the heavy oil is decomposed in the heavy oil decomposition device 20 as learning data.

In step S102, the learning unit 134 learns the second model and the third model using the acquired learning data.

In step S103, the learning unit 134 stores the learned second model and third model in the model DB 122, and ends the process.

### <3.2. PREDICTION PROCESSING>

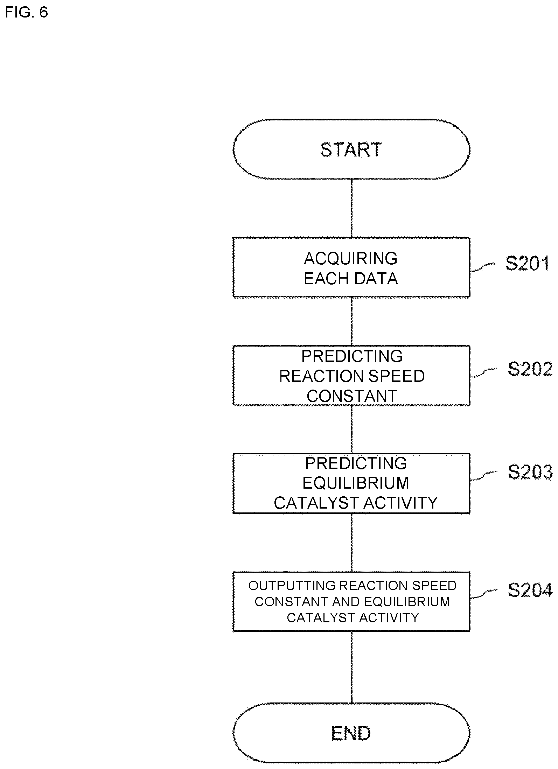

FIG. 6 is a flowchart showing an example of the flow of prediction processing performed by the information processing device 10. The information processing device 10 executes this process by inputting the oil quality of the heavy oil and the operating parameter.

In step S201, the acquisition unit 135 acquires a third parameter that is an operating parameter regarding the heavy oil decomposition device 20 that is currently performing heavy oil decomposition, the oil quality of the heavy oil, the learned second and third model.

In step S202, the prediction unit 136 predicts the reaction speed constant by inputting the oil quality of the heavy oil and the first parameter of the third parameter into the second model.

In step S203, the prediction unit 136 predicts an index value indicating the equilibrium catalyst activity by inputting the oil quality of the heavy oil and the second parameter of the third parameter into the third model.

In step S204, the output unit 140 outputs the predicted reaction speed constant and the index value indicating the equilibrium catalyst activity to an output device, etc.

Since the information processing device 10 can execute the prediction process at any timing, it can predict in real time a value indicating the reaction state of the heavy oil decomposition device 20 in operation..

### <3.3. RELEARNING PROCESSING>

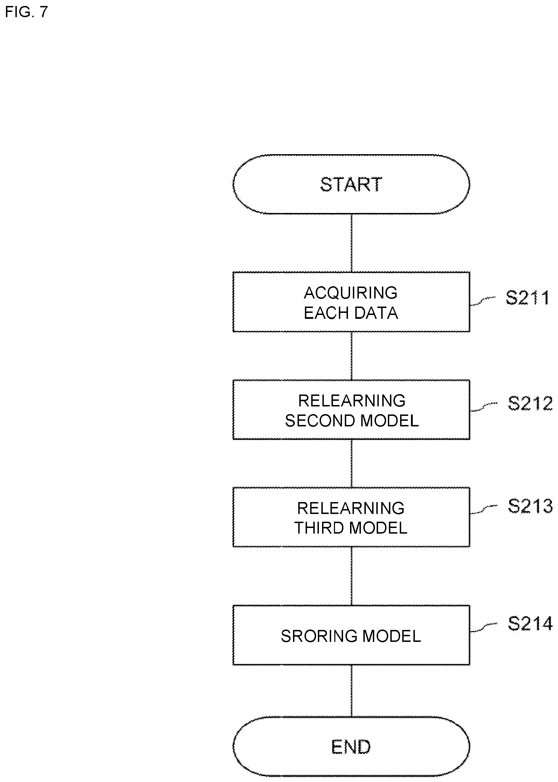

FIG. 7 is a flowchart showing an example of the flow of relearning processing performed by the information processing device 10. The information processing device 10 executes the process at an arbitrary timing (for example, when acquiring a reaction state analysis result, receiving a relearning process start signal, etc.).

In step S211, the acquisition unit 135 acquires a third parameter that is an operating parameter regarding the heavy oil decomposition device 20 that is performing heavy oil decomposition, the oil quality of the heavy oil, and the reaction state obtained by the analysis, and the learned second and third model.

In step S212, the learning unit 134 relearns the second model by using the learning data, the third parameter that is an operating parameter regarding the heavy oil decomposition device 20 that is performing heavy oil decomposition, and the analyzed reaction speed constant.

In step S213, the learning unit 134 relearns the third model by using the learning data, a third parameter that is an operating parameter regarding the heavy oil decomposition device 20 that is performing heavy oil decomposition, and an index value indicating the analyzed equilibrium catalyst activity.

In step S214, the learning unit 134 stores the relearned second and third model in the model DB 122, and ends the process.

Since the information processing device 10 can execute the prediction process at any timing, it can predict a value indicating the reaction state of the heavy oil decomposition device 20 in operation in real time.

## <3.4. PRODUCT YIELD CALCULATION PROCESSING>

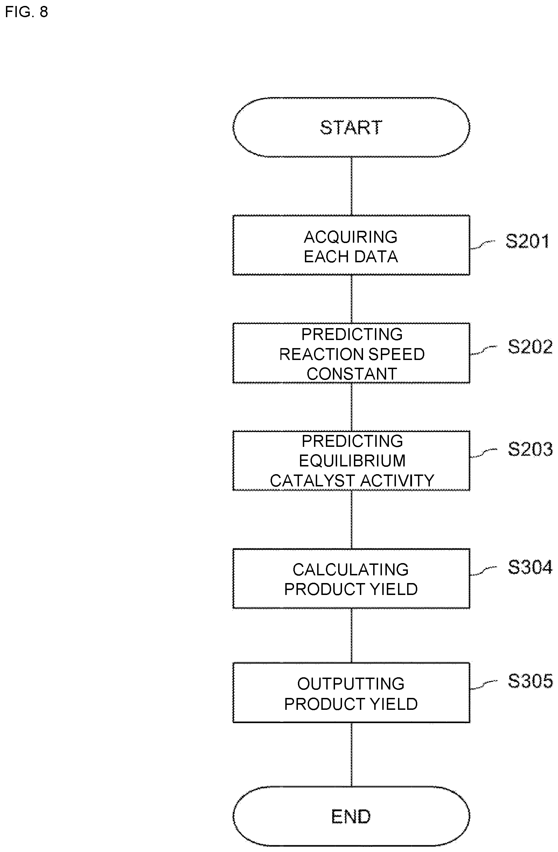

FIG. 8 is a flowchart showing an example of the flow of product yield calculation processing performed by the information processing device 10. The information processing device 10 executes the process by inputting the oil quality of the heavy oil and the operating parameter, etc. It is noted that processes that are common to the prediction process are given the same reference numerals , and the description thereof will be omitted.

In step S304, the calculation unit 138 calculates a product yield or an index value contributing to the product yield by using the oil quality of the heavy oil, the third parameter, and the value indicating the reaction state.

In step S305, the output unit 140 outputs the determined product yield or the determined index value contributing to the product yield, and ends the process.

## <3.5. OPTIMIZATION PROCESS>

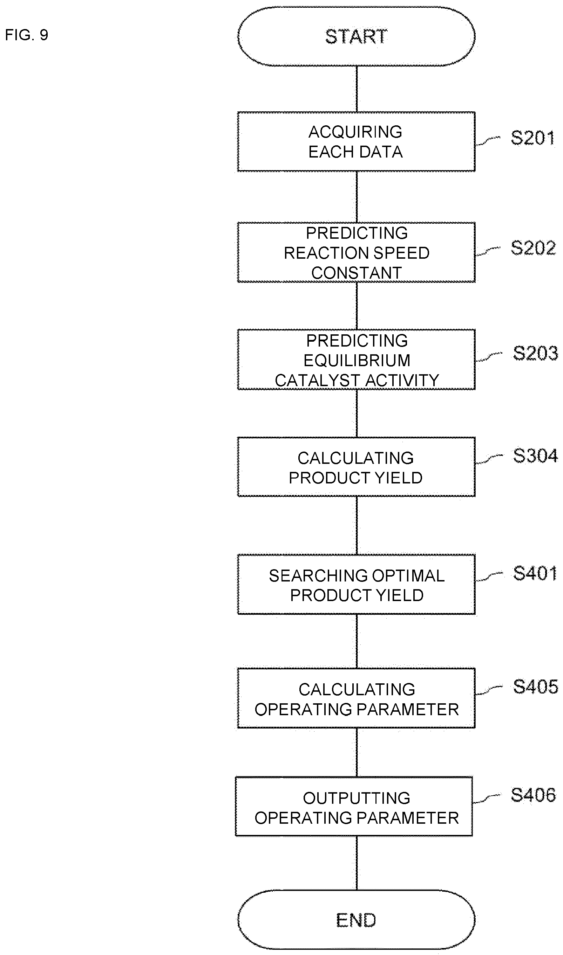

FIG. 9 is a flowchart showing an example of the flow of optimization calculation processing performed by the information processing device 10. The information processing device 10 executes the processing at an arbitrary timing. It is noted that processes that are common to the prediction process and the product yield calculation process are given the same reference numerals, and the description thereof will be omitted.

In step S401, the optimization unit 139 determines the optimal product yield by using a product yield or an index value contributing to the product yield, oil quality of the heavy oil, the first parameter and the reaction speed of the third parameter, a second parameter and an index value indicating equilibrium catalytic activity of the third parameter.

In step S405, the optimization unit 139 determines the operating parameter that achieve the determined optimal product yield by using the determined product yield or index value contributing to the product yield, oil quality of the heavy oil, operating parameter, and values indicating the reaction state.

In step S406, the output unit 140 outputs the determined optimal operating parameter, and ends the process.

As explained above, according to the present disclosure, the oil quality of the heavy oil that is the target of heavy oil decomposition, the operating parameter for the heavy oil decomposition device that performs heavy oil decomposition, and a value indicating the reaction state when the heavy oil is decomposed in the heavy oil decomposition device using operating parameter are accepted as learning data input. And the first model that outputs a reaction state of heavy oil decomposition in response to inputting the oil quality of the heavy oil and the operating parameter is learned using the learning data, the learned first model is stored in the storage unit. Thereby, it is possible to obtain a first model for accurately predicting the reaction state of heavy oil decomposition.

Further, according to the present disclosure, the third parameter that is an operating parameter regarding a heavy oil decomposition device that is performing heavy oil decomposition, oil quality of the heavy oil, and the learned first model are obtained, by inputting the oil quality of the heavy oil and the third parameter into the first model, a value indicating the reaction state is predicted, and by outputting the value indicating the reaction state, it is possible to predict the reaction state of heavy oil decomposition with high accuracy.

Further, according to the present disclosure, the first model that, in response to input of oil quality of the heavy oil that is subject to heavy oil decomposition and operating parameter related to the heavy oil decomposition device that performs heavy oil decomposition, outputs a value indicating a reaction state when the heavy oil is decomposed in the heavy oil decomposition device using the operating parameter is acquired. And, the input of the oil quality of the heavy oil and operating parameter is accepted, a value indicating the reaction state is determined using the oil quality of the heavy oil, the operating parameter, and the first model. And, the product yield or index values contributing to the product yield is determined by using the oil quality of the heavy oil, operating parameter, and values indicating the reaction state. Thereby, it is possible to improve product yield and contribute to improving refinery profits.

Further, according to the present disclosure, it is possible to improve the product yield by determining operating parameter that achieve optimal product yield using the determined product yield or index value contributing to the product yield, oil quality of the heavy oil, operating parameter, and values indicating the reaction state.

## <SECOND EMBODIMENT>

The second embodiment relates to a technique for predicting reaction states in various chemical reactions in the process of manufacturing chemical products (for example, industrial chemicals, chemical fertilizers, paper, pulp, rubber, synthetic fibers, synthetic resins, petroleum products, pharmaceuticals, dyes, detergents, cosmetics, bio products), etc. (hereinafter simply referred to as chemicals, etc.). The technique according to the second embodiment will be described as a technique for predicting a value indicating a reaction state when a chemical reaction is performed in the reaction device based on the operating parameter related to the reaction device that performs a predetermined chemical reaction and information regarding the chemical substances used in the chemical reaction in the reaction device.

Reaction models that model chemical reactions of conventional chemicals include items that are difficult to quantify, such as mass transfer. These values are determined by referring to existing papers or by experiment. For this reason, it takes a lot of time to create a reaction model, and furthermore, in a physical model such as a reaction model, the number of parameter that humans can consider is limited.

For this reason, there was a problem where variation in parameter that are not considered in the reaction model leads to a decrease in prediction accuracy.

The technology of the present disclosure prepares a model that learned the relationship between parameter of a reaction state of a chemical, etc., operating parameter, and information regarding the chemical substance. Parameter of the reaction state include, for example, parameter related to the reaction speed of chemical substances, index values indicating catalytic activity, etc. Thereby, the technology of the present disclosure can accurately predict the reaction state related to the production of chemical products, etc.

Further, the technology of the present disclosure calculates parameter of an apparent reaction state depending on operating parameter and chemical substances by using past chemical reaction results, experimental results, etc. The technology of the present disclosure learns the model by using parameter of this apparent reaction state. As a result, it is possible to accurately quantify the influence of each operational parameter that is difficult to quantify in a data-driven manner, contributing to speeding up and improving the accuracy of reaction model construction.

<4. CONFIGURATION OF INFORMATION PROCESSING SYSTEM 2>

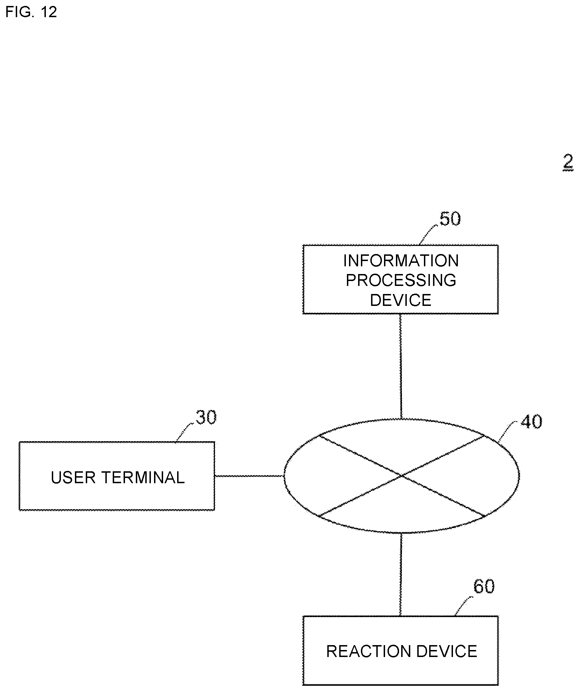

The information processing system 2 according to the present disclosure will be described using FIG. 12. The information processing system 2 according to the present disclosure is configured to include an information processing device 50, a reaction device 60, a user terminal 30, and a network 40.

<5. FUNCTIONAL CONFIGURATION OF INFORMATION PROCESSING DEVICE 50>

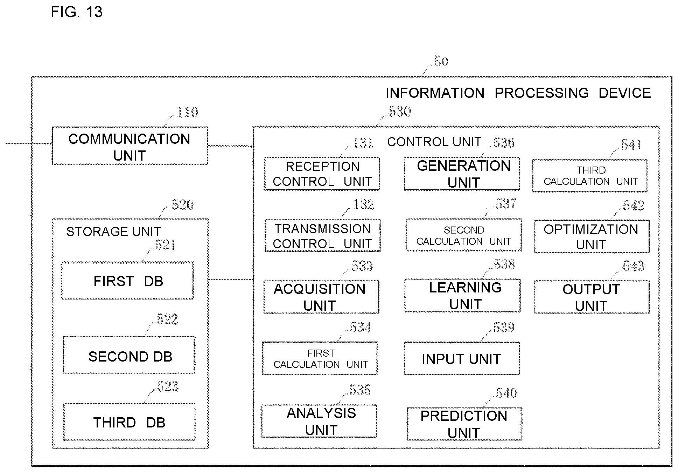

FIG. 13 is a block diagram showing the functional configuration of the information processing device 50. As shown in FIG. 13, the information processing device 50 includes a communication unit 110, a storage unit 520, and a control unit 530.

The storage unit 520 stores data and programs used by the information processing device 50. The storage unit 520 stores a first DB 521, a second DB 522, a third DB 523, etc.

The first DB 521 is a database that holds learning data used in the model learning/building process of the prediction model. The learning data is data obtained by performing the chemical reaction in the past. Specifically, the learning data is a set of real data consisting of information regarding the substance to be subjected to a chemical reaction, operating parameter regarding the reaction device 60 that performs a predetermined chemical reaction, parameter of the reaction state when the substance is chemically reacted in the reaction device 60 using the operating parameter, etc.

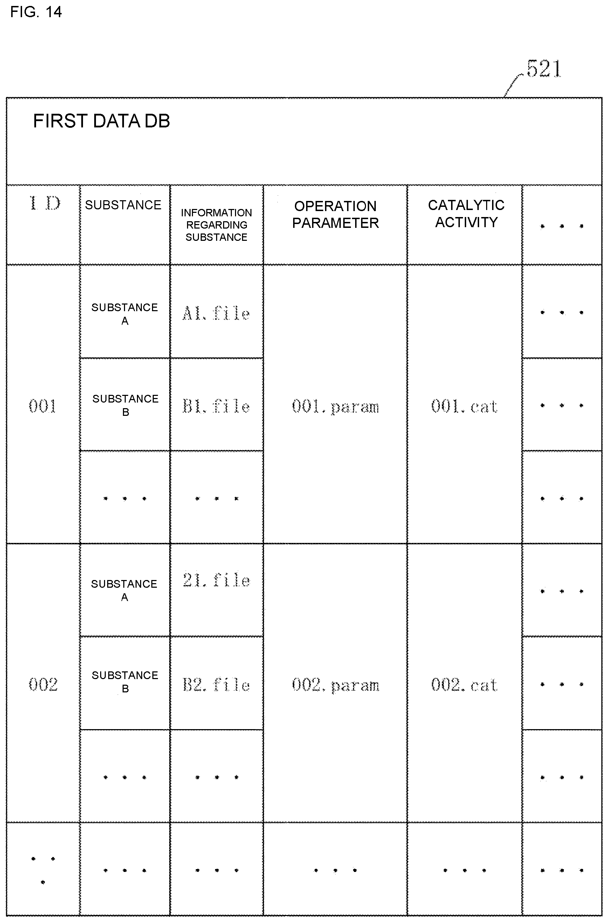

FIG. 14 is a diagram showing an example of the data structure of the first DB 521. As shown in FIG. 14, each record in the first DB 521 includes an item "ID", an item "substance", an item "quantity", an item "operating parameter", an item "catalyst activity", etc. It is noted that the items shown here are not all, and there may be no items shown here, or there may be other items.

The item "ID" stores information for identifying each record.

The item "substance" stores the name of the substance used in the chemical reaction.

The item "Information regarding substance" stores information regarding the substance used in the chemical reaction. Information regarding the substance includes, for example, the physical quality of the substance, the amount used, and the weight, etc. It is noted that when there are multiple substances, the item may also be stored including the ratio of the quantities of the substances.

The item "operating parameter" stores the operating parameter of the reaction device. The operating parameters are, for example, parameters such as the size of the reactor of the reaction device 60, the flow rate of material into the reaction device 60, the amount of catalyst, the pressure, the internal temperature, the temperature of the catalyst, the ratio of catalyst to material.

The item "catalytic activity" is data regarding the catalytic activity used in the reaction device 60 based on to the operating parameter. Data regarding catalyst activity includes the catalyst used, the amount of catalyst inflow, and an index value indicating catalyst activity, etc.

The second DB 522 stores the parameter of a reaction state that is a reaction state of a substance.

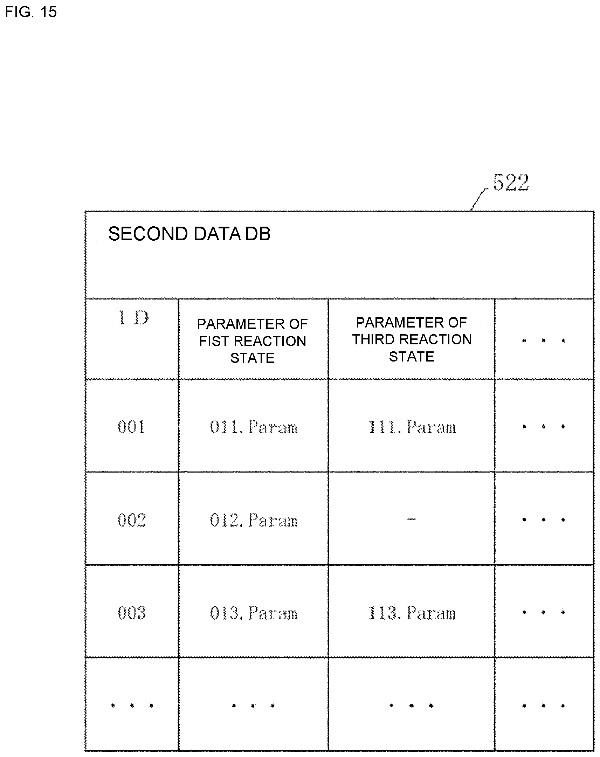

FIG. 15 is a diagram showing an example of the data structure of the second DB 522. As shown in FIG. 15, each record in the second DB 522 includes an item "ID", an item "parameter of first reaction state", an item "parameter of third reaction state", etc. It is noted that the items shown here are not all, and other items may be included.

The item "ID" stores information for identifying each record. This "ID" is linked to the "ID" of the first DB 521.

The item "parameter of first reaction state" stores parameter of a reaction state when the substance is chemically reacted in the reaction device 60 based on the operating parameter in the corresponding ID. The parameter of the first reaction state include, for example, reaction speed, reaction speed constant, frequency factor, activation energy, and catalytic activity. etc. The parameter of the first reaction state are, for example, the parameter of the first reaction state calculated by the reaction model.

The item "parameter of third reaction state" stores the parameter of the third reaction state that is the reaction state of the substance. The parameter of the third reaction state include, for example, reaction speed, reaction speed constant, frequency factor, activation energy, and catalytic activity, etc. The parameter of the third reaction state are the parameter of the third reaction state calculated by the modified model.

The third DB 523 is a database that holds various models and model parameter.

Specifically, the third DB 523 holds models used in the technology of the present disclosure, such as prediction models, reaction models, modified models, and physical models, and their parameter.

The prediction model is a model that outputs parameter of a reaction state, that is a reaction state of a substance in response to input of information regarding the substance and operating parameter. The parameter of the reaction state is, for example, a reaction speed constant of a substance when a predetermined chemical reaction is performed in the reaction device 60. Any model such as a machine learning model or a neural network can be used as the prediction model. The prediction model may represent the relationship between information about the substance, operating parameter, and reaction state of a predetermined chemical reaction, for example, by using a linear regression model.

A reaction model is a physical model that determines the parameter of a reaction state. The reaction model is, for example, a predetermined reaction kinetics model.

The modified model is a model obtained by modifying the reaction model. The modified model will be described later.

The yield model is a model that outputs a product yield or an index value contributing to the product yield by inputting information regarding substances, operating parameter, and reaction speed constants.

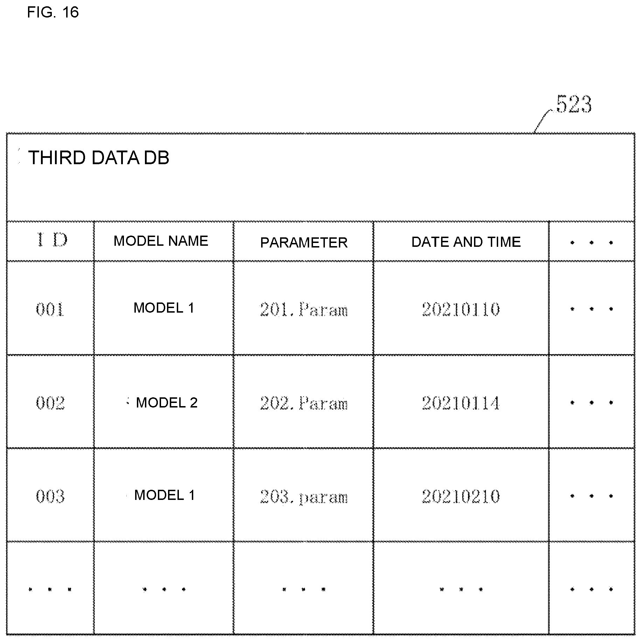

FIG. 16 is a diagram showing an example of the data structure of the third DB 523. As shown in FIG. 16, each record in the third DB 523 includes an item "ID", an item "model name", an item "parameter", an item "date and time", etc. It is noted that the items shown here are not all, and there may be no items shown here, or there may be other items.

The item "ID" stores information for identifying each record.

The item "model name" stores the name of the model.

The item "parameter" stores the parameter of the corresponding model.

The item "date and time" stores the learning date and time or acquisition date and time of the model.

The control unit 530 performs the functions shown in a reception control unit 131, a transmission control unit 132, an acquisition unit 533, a first calculation unit 534, an analysis unit 535, a generation unit 536, a second calculation unit 537, a learning unit 538, an input unit 539, a prediction unit 540, a third calculation unit 541, an optimization unit 542, an output unit 543, etc. by the processor 11 of the information processing device 50 performing processing according to the program,

The acquisition unit 533 acquires data necessary for calculating parameter of reaction state from the first DB 521.

Specifically, the acquisition unit 533 acquires a set of actual data consisting of information regarding the substance used in the chemical reaction in the reaction device 60, operating parameter of the reaction device 60, other data from when the chemical reaction was performed in the past (for example, catalyst activity, product yield, etc.), etc. from the first DB 521.

Further, the acquisition unit 533 acquires the reaction model and the parameter of the reaction model from the third DB 523.

The first calculation unit 534 calculates the parameter of a first reaction state that is predicted the reaction state of a substance when a chemical reaction is performed in the reaction device 60 with predetermined operating parameter by using information regarding the substance used in the chemical reaction in the reaction device 60 and the operating parameter of the reaction device 60.

High model prediction accuracy is required for chemical reactions in the production of chemical products. On the other hand, the parameter that can be considered by the model are limited, resulting in a large gap between the data obtained through experiments and the prediction results of the model. The technology of the present disclosure uses parameter of an apparent reaction state by determining the parameter of the first reaction state. The technology of the present disclosure aims to improve the accuracy of a prediction model by using the parameter of this apparent reaction state.

Specifically, the first calculation unit 534 first obtains information regarding the substance used in the chemical reaction in the reaction device 60 and the operating parameter of the reaction device 60 from the first DB 521. Next, the first calculation unit 534 determines, as the parameter of the first reaction state, a parameter of a first reaction rate-limiting that is predicted the reaction speed of the substance when a chemical reaction is performed in the reaction device 60 with predetermined operating parameter. More specifically, the first calculation unit 534 determines the reaction speed or reaction speed constant by using the acquired information regarding the substance used in the chemical reaction in the reaction device 60, the operating parameter of the reaction device 60, and the reaction model.

The reaction model is represented by the following formula, for example.

[Math. 2]

$$\frac{dc}{dt} = k\,c^2$$

Here, in the above formula, k is the reaction speed constant, t is time, C is the substance concentration, and $kC^2$ is the reaction speed.

Then, the first calculation unit 534 calculates, for example, k as a parameter of the first reaction speed. The calculated parameter of the first reaction state are stored in the second DB 522.

The analysis unit 535 quantifies the reaction rate-limiting by analyzing the reaction rate-limiting of the chemical reaction when the chemical reaction is performed in the reaction device 60 with predetermined operating parameter by using the correlation between the parameter of the first reaction state and the predetermined reaction rate-limiting in the chemical reaction.

Specifically, the analysis unit 535 first analyzes the reaction rate-limiting in the reaction device 60. When analyzing the reaction rate-limiting, the analysis unit 535 analyzes the reaction rate-limiting of the chemical reaction when the chemical reaction is performed in the reaction device 60 with the operating parameter by using the correlation between the parameter of the first reaction state and each of a plurality of factors that affect the parameter of the first reaction state in a chemical reaction.

The reaction rate-limiting is something that affects the determination of parameter of the reaction state among the steps that make up a reaction. The reaction rate-limiting is, for example, the one that takes the longest time in each step constituting the reaction that determines the reaction speed. Reaction rate-limiting can be expressed by multiple factors that affect the parameter of the first reaction state. Such factors include, for example, the true reaction speed, the speed of substance diffusion in the solvent, the catalyst adsorption equilibrium, the catalyst activity, etc. The analysis unit 535 analyzes the correlation between the parameter of the first reaction state that is the parameter of the apparent reaction state, and the numerical value derived from the formula of each of the plurality of factors.

Here, the correlation between the parameter of the first reaction state and each of the plurality of factors is defined as reaction speed dependence if it is a true reaction speed constant, substance diffusion dependence if it is the speed of substance diffusion in the solvent, catalyst adsorption dependent if it is catalyst adsorption equilibrium, and catalyst activity dependence if it is catalytic activity.

Next, the analysis unit 535 quantifies each of the multiple factors that influence the analyzed parameter of the first reaction state. Specifically, for example, in the case of catalyst activity rate-limiting, the analysis unit 535 determines a linear approximation equation between the apparent reaction speed constant and the catalyst activity by using data on different catalyst activities for the operating parameter and the correlation between each catalyst activity and the apparent reaction speed constant. The analysis unit 535 can quantify the rate-limiting of catalyst activity in the chemical reaction by determining the catalyst activity at the operating parameter using the determined linear approximation equation.

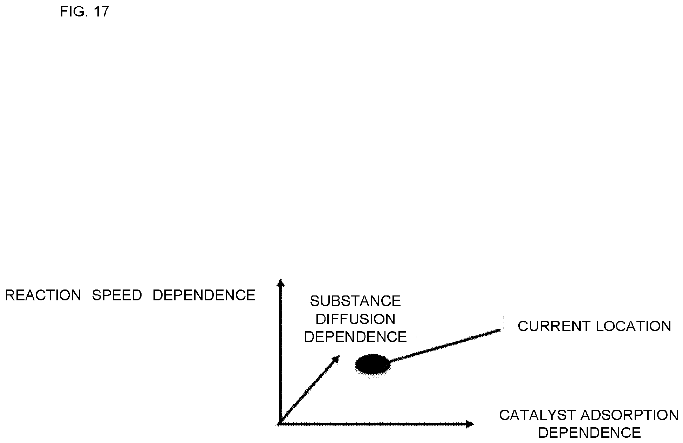

FIG. 17 is a diagram showing an example of what is the reaction rate-limiting depending on the operating state. FIG. 17 shows the relationship between reaction speed dependence, substance diffusion dependence, and catalyst adsorption dependence in operating parameter. As shown in FIG. 17, when the analysis unit 535 analyzes and quantifies the relationship between the parameter of the first reaction state and each of the multiple factors that influence the parameter of the first reaction state, its position relative to other reaction rate-limiting is visualized regarding the reaction speed of the substance in the operating parameter.

It is noted that the analysis unit 535 may be configured to display a graph quantifying the reaction rate-limiting on an output device such as a screen.

The generation unit 536 generates a modified model where a reaction model representing a chemical reaction is modified by using each of the multiple factors that influence the quantified parameter of the first reaction state.

Specifically, the generation unit 536 generates a modified model where the correlation is reflected in the reaction model using each of a plurality of factors that influence the quantified parameter of one reaction state. For example, if the catalyst activity coefficient $\alpha$ that corrects k was determined by quantifying the catalyst activity dependence, the generation unit 536 modifies the reaction model by substituting $\alpha k$' for k in the equation of the reaction model. Similarly, the generation unit 536 modifies the reaction model by substituting a predetermined equation for the substance concentration C based on substance diffusion dependence, etc.

The generation unit 536 then stores the generated modified model in the third DB 523.

The second calculation unit 537 determines the parameter of a third reaction state that predicted the reaction state of the substance when a chemical reaction is performed in the reaction device 60 using the operating parameter, by using the modified model.

Specifically, the second calculation unit 537 determines, as a parameter of the third reaction state, a parameter of a third reaction speed that is a prediction of the reaction rate of the substance when a chemical reaction is performed in the reaction device 60 with predetermined operating parameter. More specifically, the second calculation unit 537 calculates the reaction speed or reaction speed constant as a parameter of the third reaction state by using the acquired operating parameter, information regarding the substance, parameter of the first reaction state, and the modified model. Then, the second calculation unit 537 stores the calculated parameter of the third reaction state in the second DB 522. It is noted that the parameter of the third reaction state is also a parameter of an apparent reaction state.

The learning unit 538 learns a prediction model that outputs parameter of a second reaction state that is a reaction state of the substance in response to inputting information regarding the substance and operating parameter by using the information regarding the substance stored in the first DB 521 and the acquired operating parameter, and parameter of the apparent reaction state (parameter of first reaction state or parameter of third reaction state) calculated and stored in the second DB 522 as learning data.

Specifically, the learning unit 538 learns a prediction model that outputs a parameter of a second reaction rate as a parameter of a second reaction state in response to inputting information regarding the substance and operating parameter by using the information regarding the substance stored in the first DB 521, the acquired operating parameter, and the determined parameter of apparent reaction state (parameter of first reaction state or parameter of third reaction state) as learning data.

The learning unit 538 can use the parameter of the first reaction state or the parameter of the third reaction state for learning as the parameter of the apparent reaction state. The parameter of the third reaction state apply to the reaction model the influence of each of the multiple factors that affect the parameter of the first reaction state that are difficult to quantify. Therefore, the parameter of the third reaction state can contribute to improving the learning accuracy of the prediction model more than the parameter of the first reaction state. Furthermore, since a modified model is not created using the parameter of the first reaction state, it is suitable when it is desired to obtain a more accurate prediction model more quickly. Which parameter the learning unit 538 uses for learning can be arbitrarily determined.

The learning unit 538 learns the prediction model using the same learning method as in the first embodiment. By learning the prediction model by the learning unit 538, the prediction model can learn the correlation between the reaction speed or reaction speed constant that changes with changes in the substance and the operating parameter. Therefore, by using the learned prediction model, it is possible to accurately predict the reaction speed or reaction speed constant in actual operation.

The learning unit 538 then stores the learned prediction model in the third DB 523.

The input unit 539 accepts input of information regarding substances and operating parameter.

Specifically, the input unit 539 accepts input of information regarding a substance where parameter of the reaction state of the substance in the reaction device 60 are desired to determined, and operating parameter in the reaction state that is desired to determine.

The prediction unit 540 determines the parameter of the second reaction state using the information regarding the substance, the operating parameter, and the prediction model.

Specifically, the prediction unit 540 first acquires the learned prediction model from the third DB 523.

Next, the prediction unit 540 determines the parameter of the second reaction state by inputting the input information regarding the substance and the operating parameter into the prediction model.

The third calculation unit 541 determines a product yield or an index value contributing to the product yield by using information regarding the substance, operating parameter, and parameter of the second reaction state.

Specifically, the third calculation unit 541 first acquires a yield model from the third DB 523. Next, the third calculation unit 541 determines the product yield or the index value contributing to the product yield by using the information regarding the substance, the operating parameter, the parameter of the second reaction state, and the yield model. In the second embodiment, the product yield is, for example, the yield of chemicals etc. obtained by the reaction device 60. Further, the index values contributing to the product yield include operating parameter necessary for determining the product yield. The index values include, for example, reaction temperature, reaction time, catalyst concentration, reaction pressure, and substrate concentration of solvent.

The optimization unit 542 determines operating parameter that achieve optimal product yield by using the determined product yield or index value contributing to the product yield, information regarding the substance, operating parameter, and parameter of the second reaction state.

Specifically, the optimization unit 542 first determines the optimal product yield by using the product yield or the index value contributing to the product yield, information regarding heavy substances, operating parameter, and parameter of the second reaction state. For example, the optimization unit 542 searches for the optimal product yield by inputting the product yield or the index value contributing to the product yield, information regarding the substance, operating parameter, and parameter of the second reaction state into a model for searching for the optimal product yield.

Next, the optimization unit 542 determines optimal operating parameter by using the product yield or the index value contributing to the product yield, information regarding the substance, parameter of the second reaction state, and the determined optimal product yield. It is noted that the optimization unit 542 may be configured to determine optimal operating parameter by also using the learned prediction model.

The output unit 543 outputs optimal operating parameter. The output unit 543 can output using the same method as the output unit 140. Further, the output unit 543 may also be configured to output the predicted parameter of the second reaction state, the determined product yield or the index value contributing to the product yield.

It is noted that in the second embodiment, the catalyst activity may be determined, or the product yield, etc. may be determined using the determined catalyst activity by using the technology of the first embodiment.

<6. OPERATION>

Hereinafter, processing in the information processing device 50 will be explained with reference to the drawings.

<6.1. LEARNING PROCESSING>

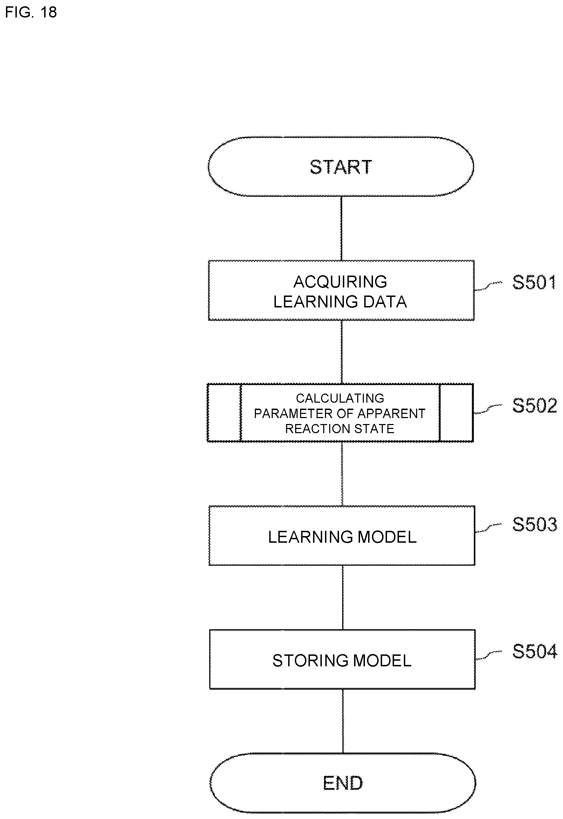

FIG. 18 is a flowchart showing an example of the flow of learning processing performed by the information processing device 50. The information processing device 50 executes this process at an arbitrary timing (for example, upon receiving a learning process start signal).

In the step S501, the acquisition unit 533 acquires information regarding substances used in a chemical reaction and operating parameter of the reaction device from the first DB 521.

In the step S502, the information processing device 50 executes a process of calculating parameter of the apparent reaction state.

In the step S503, the learning unit 538 learns the prediction model that outputs parameter of the second reaction state that is a reaction state of the substance in response to inputting information regarding the substance and operating parameter, by using the acquired information regarding the substance, the acquired operating parameter, and the determined parameter of the apparent reaction state as learning data.

In the step S504, the learning unit 538 stores the learned prediction model in the third DB 523, and ends the process.

<6.2. CALCULATION PROCESSING IN STEP S502>

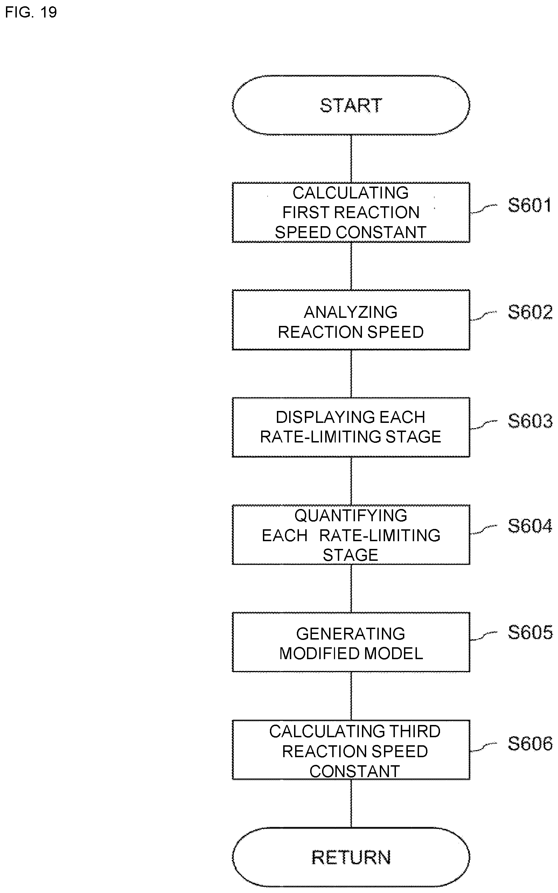

FIG. 19 is a flowchart showing an example of the flow of the calculation processing in the step S502 by the information processing device 50.

In the step S601, the first calculation unit 534 determines parameter of the first reaction state predicted as a reaction state of a substance when a chemical reaction is performed in the reaction device 60 with predetermined operating parameter by using the obtained information on the substances used in the chemical reaction and the operating parameter of the reaction device.

In the step S602, the analysis unit 535 analyzes the reaction rate-limiting in the reaction device 60 by analyzing the acquired data.

In the step S603, the analysis unit 535 displays a graph quantifying the reaction rate-limiting on an output device such as a screen.

In the step S604, the analysis unit 535 quantifies each analyzed reaction rate-limiting.

In the step S605, the generation unit 536 generates a modified model that is a modified reaction model representing a chemical reaction by using each of the plurality of factors that influence the quantified parameter of the first reaction state.

In the step S606, the second calculation unit 537 determines parameter of the third reaction state predicted by using the modified model as the reaction state of the substance when a chemical reaction is performed with the operating parameter in the reaction device 60, and returns.

<6.3. PREDICTION PROCESSING >

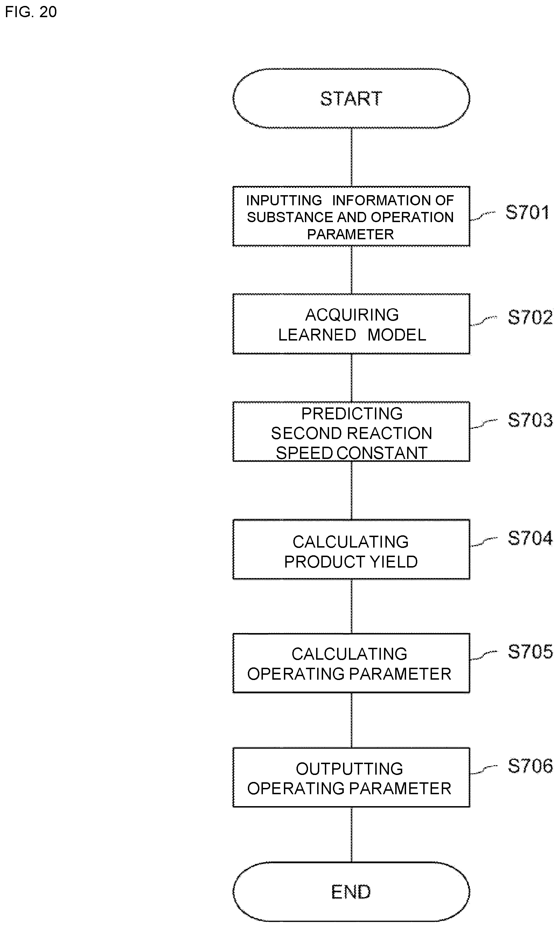

FIG. 20 is a flowchart showing an example of the flow of prediction processing performed by the information processing device 50. The information processing device 50 executes the processing by inputting information regarding the substance and operating parameter.

In the step S701, the input unit 539 accepts input of information regarding substances and operating parameter.

In the step S702, the prediction unit 540 acquires the learned prediction model from the third DB 523.

In the step S703, the prediction unit 540 determines the parameter of the second reaction state by inputting the input information regarding the substance and the operating parameter into the prediction model.

In the step S704, the third calculation unit 541 determines the product yield or the index value contributing to the product yield by using information regarding the substance, operating parameter, and parameter of the second reaction state.

In the step S705, the optimization unit 542 determines operating parameter that achieve the optimum product yield by using the determines product yield or index value contributing to the product yield, information regarding the substance, operating parameter, and parameter of the second reaction state.

In the step S706, the output unit 543 outputs the optimal operating parameter, and ends the process.

<7. SUMMRY>

As explained above, with the technology of the present disclosure, operating parameter regarding a reaction device that performs a predetermined chemical reaction and information regarding substances used in the chemical reaction in the reaction device are acquired. The parameter of a first reaction state predicted as the reaction state of the substance when a chemical reaction is performed in the reaction device using the operating parameter is determined. In the technology of the present disclosure, a prediction model that outputs parameter of a second reaction state that is a reaction state of the substance in response to inputting information regarding the substance and operating parameter is learned by using acquired information regarding the substance, acquired operating parameter, and parameter of the first reaction state as learning data. In the technology of the present disclosure, a learned prediction model is stored in a storage unit. Thereby, in the technology of the present disclosure, it is possible to learn with high accuracy a model that can predict reaction state related to the production of chemical products (e.g., industrial chemicals, chemical fertilizers, paper, pulp, rubber, synthetic fibers, synthetic resins, petroleum products, pharmaceuticals, dyes, detergents, cosmetics, bioproducts), etc.

Further, in the technology of the present disclosure, a reaction rate-limiting is quantified by analyzing the reaction rate-limiting of the chemical reaction when the chemical reaction is performed with operating parameter in the reaction device by using the correlation between the parameter of the first reaction state and the predetermined reaction rate in the chemical reaction. In the technology of the present disclosure, a modified model is generated by modifying a reaction model showing a chemical reaction by using the quantified reaction rate-limiting, parameter of a third reaction state are predicted to determine as the reaction state of the substance when a chemical reaction is performed in the reaction device with the operating parameter by using the modified model. In the technology of the present disclosure, a prediction model is learned by using the acquired information regarding the substance, the acquired operating parameter, and the parameter of the third reaction state as learning data. As a result, in the technology of the present disclosure, parameter of an apparent reaction state according to operating parameter and chemical substances are calculated by using past chemical reaction results, experimental results, etc. In the technology of the present disclosure, the model is learned by using parameter of this apparent reaction state. This makes it possible to accurately quantify in a data-driven manner the influence of each of multiple factors that affect the parameter of the first reaction state that are difficult to quantify, contributing to faster and more accurate construction of the reaction model.

<8. OTHERS>

Although the disclosed embodiments were described above, they can be implemented in various other forms, and can be implemented with various omissions, substitutions, and changes. These embodiments and modifications, as well as omissions, substitutions, and changes, are included within the technical scope of the claims and their equivalents.

For example, each function of the information processing device 10 and the information processing device 50 may be configured in another device. For example, each DB of the storage unit 120 may be constructed as an external database. Further, the technology of the first embodiment and the technology of the second embodiment can be combined as appropriate.

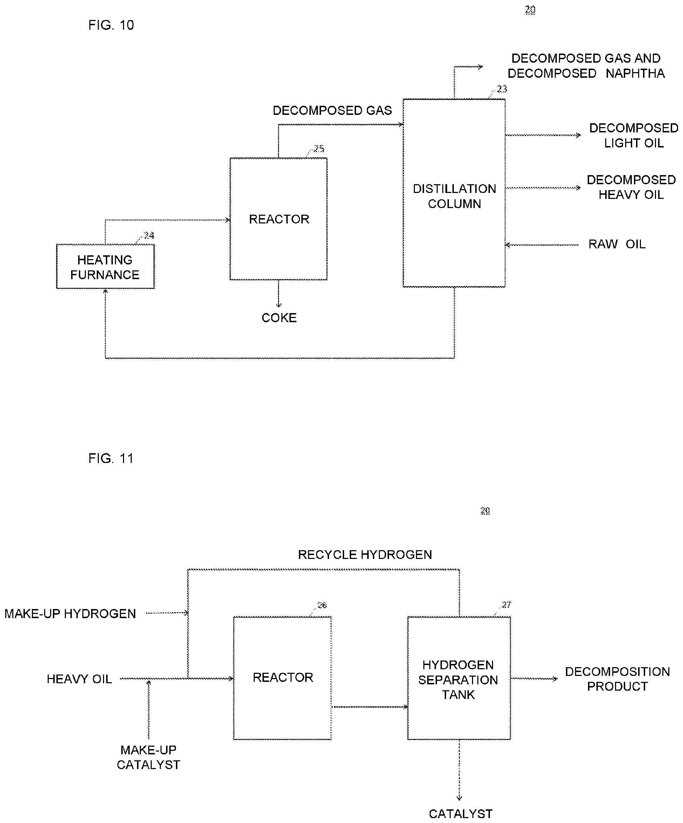

Further, in the above disclosure, the case where the heavy oil decomposition device 20 adopts FCC was described as an example, but the disclosure is not limited to this. The configuration of the present disclosure can be applied even when the heavy oil decomposition device 20 performs thermal decomposition and hydrocracking. FIG. 10 and FIG. 11 show an example of a configuration where the heavy oil decomposition device 20 performs thermal decomposition and hydrocracking. In addition, the following configuration example is an example for explanation, and does not limit the heavy oil decomposition device 20 for thermal decomposition and hydrocracking.

FIG. 10 is a diagram showing an example of the configuration of the heavy oil decomposition device 20. The example in FIG. 10 is a case where the heavy oil decomposition device 20 decomposes the heavy oil by thermal decomposition. As shown in FIG. 10, the heavy oil decomposition device 20 includes a distillation column 23, a heating furnace 24, and a reactor 25. The heavy oil decomposition device 20 has a function of controlling a distillation column 23, a heating furnace 24, and a reactor 25 during thermal decomposition, and a function of transmitting and receiving predetermined information with the information processing device 10 through communication.

The distillation column 23 receives the heavy oil as a raw material, separates each component of the heavy oil (light gas, naphtha fraction, middle distillate, each component up to heavy components, and coke) by distillation according to their boiling points, and outputs the separated components. The distillation column 23 passes the heavy oil outputted from the bottom of the distillation column 23 to the heating furnace 24 among the output components.

The heating furnace 24 heats the heavy oil separated by the distillation column 23 to a temperature necessary for thermal decomposition, and outputs the high temperature heavy oil. The heating furnace 24 passed the output high temperature heavy oil to the reactor 25.

The reactor 25 decomposes the high temperature heavy oil heated by the heating furnace 24 and outputs decomposed gas and coke. The reactor 25 performs batch cycle operation or semi-batch cycle operation. After the reaction, the reactor 25 is warmed up in preparation for the next reaction after purging of heavy oil remaining inside the reactor 25 and decoking to remove coke adhering to the surface of the reactor 25.

FIG. 11 is a diagram showing an example of the configuration of the heavy oil decomposition device 20. The example in FIG. 11 is a case where the heavy oil decomposition device 20 decomposes the heavy oil by hydrocracking. As shown in FIG. 11, the heavy oil decomposition device 20 includes a reactor 26 and a hydrogen separation tank 27. The heavy oil decomposition device 20 has a function of controlling a reactor 26 and a hydrogen separation tank 27 in hydrocracking, and a function of transmitting and receiving predetermined information with the information processing device 10 through communication.

The reactor 26 is a device for causing a decomposition reaction by bringing the heavy oil, that is a raw material, into contact with a catalyst and hydrogen, and obtaining decomposition product (light gas, naphtha fraction, middle distillate, various components up to heavy components, coke, sludge components, etc.). Specifically, when heavy oil, hydrogen, and a catalyst are introduced into the reactor 26, the reactor 26 brings the heavy oil into contact with hydrogen and the catalyst. Next, the reactor 26 obtains a decomposition product of heavy oil decomposed by a decomposition reaction when the heavy oil is brought into contact with hydrogen and a catalyst. The reactor 26 then outputs the decomposed oil.

The hydrogen separation tank 27 separates the decomposition products decomposed by the reactor 26 and hydrogen (including the catalyst in the case of slurry bed hydrocracking). Specifically, the hydrogen separation tank 27 includes a high-pressure hydrogen separation tank, a medium-pressure hydrogen separation tank, and a low-pressure hydrogen separation tank (including a catalyst separation tank in the case of slurry bed hydrocracking), and separates the decomposition products and hydrogen (in the case of slurry bed hydrocracking, the catalyst is also separated) in each tank. The hydrogen separation tank 27 outputs the separated decomposition products and hydrogen (catalyst in the case of slurry bed hydrocracking).

Further, in the second embodiment, an example was given regarding the production of chemical products, but the present disclosure is not limited to this. It is clear that the technology of the present disclosure can be applied to the production of products that involve chemical reactions, particularly when such production is performed in a plant.

**Claims**

1. An information processing device (10, 50) including a processor (11), wherein

the information processing device (10, 50) executes a step of acquiring an operating parameter regarding a reaction device (60) that performs a predetermined chemical reaction in a process of manufacturing a product and information regarding a substance used in the chemical reaction in the reaction device (60),
the information processing device (10, 50) executes a step of determining a parameter of a first reaction speed predicted as a reaction speed of the substance when the chemical reaction is performed with the operating parameter in the reaction device (60),
the information processing device (10, 50) executes a step of quantifying at least one of a plurality of factors by analyzing each of the plurality of factors of the chemical reaction when the chemical reaction is performed with the operating parameter in the reaction device (60) by using a correlation between the parameter of the first reaction speed and each of a plurality of factors that affect the parameter of the first reaction speed in the chemical reaction,
the information processing device (10, 50) executes a step of generating a modified model by modifying a reaction model representing the chemical reaction by using each of the plurality of quantified factors,
the information processing device (10, 50) executes a step of determining a parameter of a third reaction speed predicted as a reaction speed of the substance when the chemical reaction is performed with the operating parameter in the reaction device (60) by using the modified model,
**characterized in that**

the information processing device (10, 50) executes a step of learning a prediction model that outputs a parameter of a second reaction speed that is a reaction speed of the substance in response to inputting information regarding the substance and the operating parameter, by using the acquired information regarding the substance, the acquired operating parameter and the parameter of a reaction speed as learning data,
the information processing device (10, 50) executes a step of storing the learned prediction model in a storage unit (120, 520), and
the step of learning is a step of selectively learning the parameter of the first reaction speed or the parameter of the third reaction speed as a parameter of a reaction speed.

2. The information processing device (10, 50) of Claim 1, wherein
the step of quantifying step is a step of quantifying each of the plurality of factors including at least two of a true reaction speed, a speed of substance diffusion, a catalyst adsorption equilibrium and a catalyst activity.

3. An information processing device (10, 50) including a processor (11), wherein

the information processing device (10, 50) executes a step of acquiring the prediction model learned based on the parameter of the first reaction speed or the parameter of the third reaction speed by the information processing device (10, 50) of Claim 1 or 2,
the information processing device (10, 50) executes a step of accepting input of information regarding the substance and the operating parameter,
the information processing device (10, 50) executes a step of determining a parameter of the second reaction speed by using information regarding the substance, the operating parameter and the prediction model,
the information processing device (10, 50) executes a step of determining a product yield or an index value contributing the product yield by using information regarding the substance, the operating parameter and the parameter of the second reaction speed, and
the information processing device (10, 50) executes a step of outputting the determined product yield or index value contributing the product yield.

4. The information processing device (10, 50) of Claim 1 or 2, wherein

the information processing device (10, 50) executes a step of relearning the prediction model based on an analysis result in response to acquiring the analysis result of an analyzed reaction speed of the substance when the chemical reaction is performed with the operating parameter in the reaction device (60), and
the information processing device (10, 50) executes a step of storing the relearned prediction model in a storage unit (120, 520).

5. The information processing device (10, 50) of Claim 1 or 2, wherein
the prediction model is capable of outputting in real time a parameter of the second reaction speed that is a reaction speed of the substance in response to inputting information regarding the substance and the operating parameter.

6. A method for a computer including a processor (11) to execute:

a step of acquiring an operating parameter regarding a reaction device (60) that performs a predetermined chemical reaction in the process of manufacturing a product and information regarding a substance used in the chemical reaction in the reaction device (60);
a step of determining a parameter of a first reaction speed predicted as a reaction speed of the substance when the chemical reaction is performed with the operating parameter in the reaction device (60);
a step of quantifying at least one of a plurality of factors by analyzing each of the plurality of factors of the chemical reaction when the chemical reaction is performed with the operating parameter in the reaction device (60) by using a correlation between the parameter of the first reaction speed and each of a plurality of factors that affect the parameter of the first reaction speed in the chemical reaction;
a step of generating a modified model by modifying a reaction model representing the chemical reaction by using each of the plurality of quantified factors;
a step of determining a parameter of a third reaction speed predicted as a reaction speed of the substance when the chemical reaction is performed with the operating parameter in the reaction device (60) by using the modified model;
**characterized by**
a step of learning a prediction model that outputs a parameter of a second reaction speed that is a reaction speed of the substance in response to inputting information regarding the substance and the operating parameter, by using the acquired information regarding the substance, the acquired operating parameter and the parameter of a reaction speed as learning data; and

a step of storing the learned prediction model in a storage unit (120, 520), wherein

the step of learning is a step of selectively learning the parameter of the first reaction speed or the parameter of the third reaction speed as a parameter of a reaction speed.

7. A program causing a processor (11) included in a computer to execute:

a step of acquiring an operating parameter regarding a reaction device (60) that performs a predetermined chemical reaction in the process of manufacturing a product and information regarding a substance used in the chemical reaction in the reaction device (60);

a step of determining a parameter of a first reaction speed predicted as a reaction speed of the substance when the chemical reaction is performed with the operating parameter in the reaction device (60);

a step of quantifying at least one of a plurality of factors by analyzing each of the plurality of factors of the chemical reaction when the chemical reaction is performed with the operating parameter in the reaction device (60) by using a correlation between the parameter of the first reaction speed and each of a plurality of factors that affect the parameter of the first reaction speed in the chemical reaction;

a step of generating a modified model by modifying a reaction model representing the chemical reaction by using each of the plurality of quantified factors;

a step of determining a parameter of a third reaction speed predicted as a reaction speed of the substance when the chemical reaction is performed with the operating parameter in the reaction device (60) by using the modified model;

**characterized by**

a step of learning a prediction model that outputs a parameter of a second reaction speed that is a reaction speed of the substance in response to inputting information regarding the substance and the operating parameter, by using the acquired information regarding the substance, the acquired operating parameter and the parameter of a reaction speed as learning data; and

a step of storing the learned prediction model in a storage unit (120, 520), wherein

the step of learning is a step of selectively learning the parameter of the first reaction speed or the parameter of the third reaction speed as a parameter of a reaction speed.

**Patentansprüche**

1. Informationsverarbeitungsvorrichtung (10, 50), die einen Prozessor (11) beinhaltet, wobei

die Informationsverarbeitungsvorrichtung (10, 50) einen Schritt zum Erfassen eines Betriebsparameters bezüglich einer Reaktionsvorrichtung (60), die eine vorbestimmte chemische Reaktion in einem Prozess zum Herstellen eines Produkts durchführt, und von Informationen bezüglich einer Substanz, die in der chemischen Reaktion in der Reaktionsvorrichtung (60) verwendet wird, ausführt,

die Informationsverarbeitungsvorrichtung (10, 50) einen Schritt zum Bestimmen eines Parameters einer ersten Reaktionsgeschwindigkeit ausführt, die als eine Reaktionsgeschwindigkeit der Substanz vorhergesagt wird, wenn die chemische Reaktion mit dem Betriebsparameter in der Reaktionsvorrichtung (60) durchgeführt wird,

die Informationsverarbeitungsvorrichtung (10, 50) einen Schritt zum Quantifizieren von mindestens einem aus einer Vielzahl von Faktoren durch Analysieren jedes der Vielzahl von Faktoren der chemischen Reaktion ausführt, wenn die chemische Reaktion mit dem Betriebsparameter in der Reaktionsvorrichtung (60) durchgeführt wird, indem eine Korrelation zwischen dem Parameter der ersten Reaktionsgeschwindigkeit und jedem aus einer Vielzahl von Faktoren verwendet wird, die den Parameter der ersten Reaktionsgeschwindigkeit in der chemischen Reaktion beeinflussen,

die Informationsverarbeitungsvorrichtung (10, 50) einen Schritt zum Erzeugen eines modifizierten Modells durch Modifizieren eines Reaktionsmodells ausführt, das die chemische Reaktion unter Verwendung jedes der Vielzahl von quantifizierten Faktoren darstellt,

die Informationsverarbeitungsvorrichtung (10, 50) einen Schritt zum Bestimmen eines Parameters einer dritten Reaktionsgeschwindigkeit ausführt, die als eine Reaktionsgeschwindigkeit der Substanz vorhergesagt wird, wenn die chemische Reaktion mit dem Betriebsparameter in der Reaktionsvorrichtung (60) unter Verwendung des modifizierten Modells durchgeführt wird,

**dadurch gekennzeichnet, dass**

die Informationsverarbeitungsvorrichtung (10, 50) einen Schritt zum Lernen eines Vorhersagemodells ausführt, das einen Parameter einer zweiten Reaktionsgeschwindigkeit, die eine Reaktionsgeschwindigkeit der Substanz ist, als Reaktion auf Eingeben von Informationen bezüglich der Substanz und des Betriebsparameters ausgibt, indem die erfassten Informationen bezüglich der Substanz, der erfasste Betriebsparameter und der Parameter einer Reaktionsgeschwindigkeit als Lerndaten verwendet werden, die Informationsverarbeitungsvorrichtung (10, 50) einen Schritt zum Speichern des gelernten Vorhersagemodells in einer Speichereinheit (120, 520) ausführt, und der Schritt zum Lernen ein Schritt zum selektiven Lernen des Parameters der ersten Reaktionsgeschwindigkeit oder des Parameters der dritten Reaktionsgeschwindigkeit als Parameter einer Reaktionsgeschwindigkeit ist.

2. Informationsverarbeitungsvorrichtung (10, 50) nach Anspruch 1, wobei
der Schritt zum Quantifizieren ein Schritt zum Quantifizieren jedes der Vielzahl von Faktoren ist, die mindestens zwei von einer tatsächlichen Reaktionsgeschwindigkeit, einer Geschwindigkeit einer Substanzdiffusion, einem Katalysatoradsorptionsgleichgewicht und einer Katalysatoraktivität beinhalten.

3. Informationsverarbeitungsvorrichtung (10, 50), die einen Prozessor beinhaltet (11), wobei

die Informationsverarbeitungsvorrichtung (10, 50) einen Schritt zum Erfassen des gelernten Vorhersagemodells basierend auf dem Parameter der ersten Reaktionsgeschwindigkeit oder dem Parameter der dritten Reaktionsgeschwindigkeit durch die Informationsverarbeitungsvorrichtung (10, 50) nach Anspruch 1 oder 2 ausführt, die Informationsverarbeitungsvorrichtung (10, 50) einen Schritt zum Akzeptieren einer Eingabe von Informationen bezüglich der Substanz und des Betriebsparameters ausführt, die Informationsverarbeitungsvorrichtung (10, 50) einen Schritt zum Bestimmen eines Parameters der zweiten Reaktionsgeschwindigkeit unter Verwendung von Informationen bezüglich der Substanz, des Betriebsparameters und des Vorhersagemodells ausführt, die Informationsverarbeitungsvorrichtung (10, 50) einen Schritt zum Bestimmen einer Produktausbeute oder eines Indexwertes, der zur Produktausbeute beiträgt, unter Verwendung von Informationen bezüglich der Substanz, des Betriebsparameters und des Parameters der zweiten Reaktionsgeschwindigkeit ausführt, und die Informationsverarbeitungsvorrichtung (10, 50) einen Schritt zum Ausgeben der bestimmten Produktausbeute oder des bestimmten Indexwertes, der zur Produktausbeute beiträgt, ausführt.

4. Informationsverarbeitungsvorrichtung (10, 50) nach Anspruch 1 oder 2, wobei

die Informationsverarbeitungsvorrichtung (10, 50) einen Schritt zum erneuten Lernen des Vorhersagemodells basierend auf einem Analyseergebnis als Reaktion auf Erfassen des Analyseergebnisses einer analysierten Reaktionsgeschwindigkeit der Substanz ausführt, wenn die chemische Reaktion mit dem Betriebsparameter in der Reaktionsvorrichtung (60) durchgeführt wird, und die Informationsverarbeitungsvorrichtung (10, 50) einen Schritt zum Speichern des erneut gelernten Vorhersagemodells in einer Speichereinheit (120, 520) ausführt.

5. Informationsverarbeitungsvorrichtung (10, 50) nach Anspruch 1 oder 2, wobei
das Vorhersagemodell in der Lage ist, in Echtzeit einen Parameter der zweiten Reaktionsgeschwindigkeit, wobei es sich um eine Reaktionsgeschwindigkeit der Substanz handelt, als Reaktion auf Eingeben von Informationen bezüglich der Substanz und des Betriebsparameters auszugeben.

6. Verfahren für einen Computer, der einen Prozessor (11) beinhaltet, um Folgendes auszuführen:

einen Schritt zum Erfassen eines Betriebsparameters bezüglich einer Reaktionsvorrichtung (60), die eine vorbestimmte chemische Reaktion in einem Prozess zum Herstellen eines Produkts durchführt, und von Informationen bezüglich einer Substanz, die in der chemischen Reaktion in der Reaktionsvorrichtung (60) verwendet wird; einen Schritt zum Bestimmen eines Parameters einer ersten Reaktionsgeschwindigkeit, die als eine Reaktionsgeschwindigkeit der Substanz vorhergesagt wird, wenn die chemische Reaktion mit dem Betriebsparameter in der Reaktionsvorrichtung (60) durchgeführt wird; einen Schritt zum Quantifizieren von mindestens einem aus einer Vielzahl von Faktoren durch Analysieren jedes der Vielzahl von Faktoren der chemischen Reaktion, wenn die chemische Reaktion mit dem Betriebsparameter in der Reaktionsvorrichtung (60) durchgeführt wird, indem eine Korrelation zwischen dem Parameter der ersten Reaktionsgeschwindigkeit und jedem aus einer Vielzahl von Faktoren verwendet wird, die den Parameter der ersten Reaktionsgeschwindigkeit in der chemischen Reaktion beeinflussen;

einen Schritt zum Erzeugen eines modifizierten Modells durch Modifizieren eines Reaktionsmodells, das die chemische Reaktion unter Verwendung jedes der Vielzahl von quantifizierten Faktoren darstellt;

einen Schritt zum Bestimmen eines Parameters einer dritten Reaktionsgeschwindigkeit, die als eine Reaktionsgeschwindigkeit der Substanz vorhergesagt wird, wenn die chemische Reaktion mit dem Betriebsparameter in der Reaktionsvorrichtung (60) unter Verwendung des modifizierten Modells durchgeführt wird;

**gekennzeichnet durch**

einen Schritt zum Lernen eines Vorhersagemodells, das einen Parameter einer zweiten Reaktionsgeschwindigkeit, die eine Reaktionsgeschwindigkeit der Substanz ist, als Reaktion auf Eingeben von Informationen bezüglich der Substanz und des Betriebsparameters ausgibt, indem die erfassten Informationen bezüglich der Substanz, der erfasste Betriebsparameter und der Parameter einer Reaktionsgeschwindigkeit als Lerndaten verwendet werden; und

einen Schritt zum Speichern des gelernten Vorhersagemodells in einer Speichereinheit (120, 520), wobei der Schritt zum Lernen ein Schritt zum selektiven Lernen des Parameters der ersten Reaktionsgeschwindigkeit oder des Parameters der dritten Reaktionsgeschwindigkeit als Parameter einer Reaktionsgeschwindigkeit ist.

7. Programm, das einen Prozessor (11), der in einem Computer enthalten ist, veranlasst, Folgendes auszuführen:

einen Schritt zum Erfassen eines Betriebsparameters bezüglich einer Reaktionsvorrichtung (60), die eine vorbestimmte chemische Reaktion in einem Prozess zum Herstellen eines Produkts durchführt, und von Informationen bezüglich einer Substanz, die in der chemischen Reaktion in der Reaktionsvorrichtung (60) verwendet wird;

einen Schritt zum Bestimmen eines Parameters einer ersten Reaktionsgeschwindigkeit, die als eine Reaktionsgeschwindigkeit der Substanz vorhergesagt wird, wenn die chemische Reaktion mit dem Betriebsparameter in der Reaktionsvorrichtung (60) durchgeführt wird;

einen Schritt zum Quantifizieren von mindestens einem aus einer Vielzahl von Faktoren durch Analysieren jedes der Vielzahl von Faktoren der chemischen Reaktion, wenn die chemische Reaktion mit dem Betriebsparameter in der Reaktionsvorrichtung (60) durchgeführt wird, indem eine Korrelation zwischen dem Parameter der ersten Reaktionsgeschwindigkeit und jedem aus einer Vielzahl von Faktoren verwendet wird, die den Parameter der ersten Reaktionsgeschwindigkeit in der chemischen Reaktion beeinflussen;

einen Schritt zum Erzeugen eines modifizierten Modells durch Modifizieren eines Reaktionsmodells, das die chemische Reaktion unter Verwendung jedes der Vielzahl von quantifizierten Faktoren darstellt;

einen Schritt zum Bestimmen eines Parameters einer dritten Reaktionsgeschwindigkeit, die als eine Reaktionsgeschwindigkeit der Substanz vorhergesagt wird, wenn die chemische Reaktion mit dem Betriebsparameter in der Reaktionsvorrichtung (60) unter Verwendung des modifizierten Modells durchgeführt wird;

**gekennzeichnet durch**

einen Schritt zum Lernen eines Vorhersagemodells, das einen Parameter einer zweiten Reaktionsgeschwindigkeit, die eine Reaktionsgeschwindigkeit der Substanz ist, als Reaktion auf Eingeben von Informationen bezüglich der Substanz und des Betriebsparameters ausgibt, indem die erfassten Informationen bezüglich der Substanz, der erfasste Betriebsparameter und der Parameter einer Reaktionsgeschwindigkeit als Lerndaten verwendet werden; und

einen Schritt zum Speichern des gelernten Vorhersagemodells in einer Speichereinheit (120, 520), wobei der Schritt zum Lernen ein Schritt zum selektiven Lernen des Parameters der ersten Reaktionsgeschwindigkeit oder des Parameters der dritten Reaktionsgeschwindigkeit als Parameter einer Reaktionsgeschwindigkeit ist.

**Revendications**

1. Dispositif de traitement d'informations (10, 50) comprenant un processeur (11), dans lequel

le dispositif de traitement d'informations (10, 50) exécute une étape d'acquisition d'un paramètre de fonctionnement concernant un dispositif de réaction (60) qui effectue une réaction chimique prédéterminée dans un processus de fabrication d'un produit et d'informations concernant une substance utilisée dans la réaction chimique dans le dispositif de réaction (60),

le dispositif de traitement d'informations (10, 50) exécute une étape de détermination d'un paramètre d'une première vitesse de réaction prédite en tant que vitesse de réaction de la substance lorsque la réaction chimique est effectuée avec le paramètre de fonctionnement dans le dispositif de réaction (60),

le dispositif de traitement d'informations (10, 50) exécute une étape de quantification d'au moins un d'une pluralité de facteurs en analysant chacun de la pluralité de facteurs de la réaction chimique lorsque la réaction chimique est effectuée avec le paramètre de fonctionnement dans le dispositif de réaction (60) en utilisant une corrélation entre le paramètre de la première vitesse de réaction et chacun d'une pluralité de facteurs qui affectent le paramètre de la première vitesse de réaction dans la réaction chimique,

le dispositif de traitement d'informations (10, 50) exécute une étape de génération d'un modèle modifié en modifiant un modèle de réaction représentant la réaction chimique en utilisant chacun de la pluralité de facteurs quantifiés,

le dispositif de traitement d'informations (10, 50) exécute une étape de détermination d'un paramètre d'une troisième vitesse de réaction prédite en tant que vitesse de réaction de la substance lorsque la réaction chimique est effectuée avec le paramètre de fonctionnement dans le dispositif de réaction (60) en utilisant le modèle modifié,

**caractérisé en ce que**

le dispositif de traitement d'informations (10, 50) exécute une étape d'apprentissage d'un modèle de prédiction qui sort un paramètre d'une deuxième vitesse de réaction qui est une vitesse de réaction de la substance en réponse à l'entrée d'informations concernant la substance et le paramètre de fonctionnement, en utilisant les informations acquises concernant la substance, le paramètre de fonctionnement acquis et le paramètre d'une vitesse de réaction en tant que données d'apprentissage,

le dispositif de traitement d'informations (10, 50) exécute une étape de stockage du modèle de prédiction appris dans une unité de stockage (120, 520), et

l'étape d'apprentissage est une étape d'apprentissage sélectif du paramètre de la première vitesse de réaction ou du paramètre de la troisième vitesse de réaction en tant que paramètre d'une vitesse de réaction.

2. Dispositif de traitement d'informations (10, 50) selon la revendication 1, dans lequel
l'étape de quantification est une étape de quantification de chacun de la pluralité de facteurs comprenant au moins deux d'une vitesse de réaction réelle, d'une vitesse de diffusion de substance, d'un équilibre d'adsorption de catalyseur et d'une activité de catalyseur.

3. Dispositif de traitement d'informations (10, 50) comprenant un processeur (11), dans lequel

le dispositif de traitement d'informations (10, 50) exécute une étape d'acquisition du modèle de prédiction appris en se basant sur le paramètre de la première vitesse de réaction ou le paramètre de la troisième vitesse de réaction par le dispositif de traitement d'informations (10, 50) selon la revendication 1 ou 2,

le dispositif de traitement d'informations (10, 50) exécute une étape d'acceptation de l'entrée d'informations concernant la substance et le paramètre de fonctionnement,

le dispositif de traitement d'informations (10, 50) exécute une étape de détermination d'un paramètre de la deuxième vitesse de réaction en utilisant des informations concernant la substance, le paramètre de fonctionnement et le modèle de prédiction,

le dispositif de traitement d'informations (10, 50) exécute une étape de détermination d'un rendement de produit ou d'une valeur d'indice contribuant au rendement de produit en utilisant des informations concernant la substance, le paramètre de fonctionnement et le paramètre de la deuxième vitesse de réaction, et

le dispositif de traitement d'informations (10, 50) exécute une étape de sortie du rendement de produit déterminé ou de la valeur d'indice contribuant au rendement de produit.

4. Dispositif de traitement d'informations (10, 50) selon la revendication 1 ou 2, dans lequel

le dispositif de traitement d'informations (10, 50) exécute une étape de réapprentissage du modèle de prédiction en se basant sur un résultat d'analyse en réponse à l'acquisition du résultat d'analyse d'une vitesse de réaction analysée de la substance lorsque la réaction chimique est effectuée avec le paramètre de fonctionnement dans le dispositif de réaction (60), et

le dispositif de traitement d'informations (10, 50) exécute une étape de stockage du modèle de prédiction réappris dans une unité de stockage (120, 520).

5. Dispositif de traitement d'informations (10, 50) selon la revendication 1 ou 2, dans lequel
le modèle de prédiction est capable de sortir en temps réel un paramètre de la deuxième vitesse de réaction qui est une vitesse de réaction de la substance en réponse à l'entrée d'informations concernant la substance et le paramètre de fonctionnement.

6. Procédé pour un ordinateur comprenant un processeur (11) pour exécuter :

une étape d'acquisition d'un paramètre de fonctionnement concernant un dispositif de réaction (60) qui effectue une réaction chimique prédéterminée dans le processus de fabrication d'un produit et des informations concernant une substance utilisée dans la réaction chimique dans le dispositif de réaction (60) ;
une étape de détermination d'un paramètre d'une première vitesse de réaction prédite en tant que vitesse de réaction de la substance lorsque la réaction chimique est effectuée avec le paramètre de fonctionnement dans le dispositif de réaction (60) ;
une étape de quantification d'au moins un d'une pluralité de facteurs en analysant chacun de la pluralité de facteurs de la réaction chimique lorsque la réaction chimique est effectuée avec le paramètre de fonctionnement dans le dispositif de réaction (60) en utilisant une corrélation entre le paramètre de la première vitesse de réaction et chacun d'une pluralité de facteurs qui affectent le paramètre de la première vitesse de réaction dans la réaction chimique ;
une étape de génération d'un modèle modifié en modifiant un modèle de réaction représentant la réaction chimique en utilisant chacun de la pluralité de facteurs quantifiés ;
une étape de détermination d'un paramètre d'une troisième vitesse de réaction prédite en tant que vitesse de réaction de la substance lorsque la réaction chimique est effectuée avec le paramètre de fonctionnement dans le dispositif de réaction (60) en utilisant le modèle modifié ;

**caractérisé par**

une étape d'apprentissage d'un modèle de prédiction qui sort un paramètre d'une deuxième vitesse de réaction qui est une vitesse de réaction de la substance en réponse à l'entrée d'informations concernant la substance et le paramètre de fonctionnement, en utilisant les informations acquises concernant la substance, le paramètre de fonctionnement acquis et le paramètre d'une vitesse de réaction en tant que données d'apprentissage ; et
une étape de stockage du modèle de prédiction appris dans une unité de stockage (120, 520), dans lequel l'étape d'apprentissage est une étape d'apprentissage sélectif du paramètre de la première vitesse de réaction ou du paramètre de la troisième vitesse de réaction en tant que paramètre d'une vitesse de réaction.

7. Programme amenant un processeur (11) inclus dans un ordinateur a exécuter :

une étape d'acquisition d'un paramètre de fonctionnement concernant un dispositif de réaction (60) qui effectue une réaction chimique prédéterminée dans le processus de fabrication d'un produit et des informations concernant une substance utilisée dans la réaction chimique dans le dispositif de réaction (60) ;
une étape de détermination d'un paramètre d'une première vitesse de réaction prédite en tant que vitesse de réaction de la substance lorsque la réaction chimique est effectuée avec le paramètre de fonctionnement dans le dispositif de réaction (60) ;
une étape de quantification d'au moins un d'une pluralité de facteurs en analysant chacun de la pluralité de facteurs de la réaction chimique lorsque la réaction chimique est effectuée avec le paramètre de fonctionnement dans le dispositif de réaction (60) en utilisant une corrélation entre le paramètre de la première vitesse de réaction et chacun d'une pluralité de facteurs qui affectent le paramètre de la première vitesse de réaction dans la réaction chimique ;
une étape de génération d'un modèle modifié en modifiant un modèle de réaction représentant la réaction chimique en utilisant chacun de la pluralité de facteurs quantifiés ;
une étape de détermination d'un paramètre d'une troisième vitesse de réaction prédite en tant que vitesse de réaction de la substance lorsque la réaction chimique est effectuée avec le paramètre de fonctionnement dans le dispositif de réaction (60) en utilisant le modèle modifié ;

**caractérisé par**

une étape d'apprentissage d'un modèle de prédiction qui sort un paramètre d'une deuxième vitesse de réaction qui est une vitesse de réaction de la substance en réponse à l'entrée d'informations concernant la substance et le paramètre de fonctionnement, en utilisant les informations acquises concernant la substance, le paramètre de fonctionnement acquis et le paramètre d'une vitesse de réaction en tant que données d'apprentissage ; et une étape de stockage du modèle de prédiction appris dans une unité de stockage (120, 520), dans lequel l'étape d'apprentissage est une étape d'apprentissage sélectif du paramètre de la première vitesse de réaction ou du paramètre de la troisième vitesse de réaction en tant que paramètre d'une vitesse de réaction.

FIG. 1

FIG. 2

FIG. 3

20

DECOMPOSED OIL ←

EXHAUST GAS →

21

22

EQUILIBRIUM
CATALYST
←

REACTOR

REGENERATOR

NEW CATALYST →

USED
CATALYST
→

STEAM →

HEAVY OIL

AIR

FIG. 4

INFORMATION PROCESSING DEVICE

COMMUNICATION
UNIT

CONTROL UNIT

RECEPTION
CONTROL UNIT

PREDICTION UNIT

STORAGE UNIT

LEARNING
DATA DB

TRANSMISSION
CONTROL UNIT

DETERMINATION
UNIT

INPUT UNIT

CALCULATION
UNIT

MODEL DB

LEARNING UNIT

OPTIMIZATION
UNIT

ACQUISITION UNIT

OUTPUT UNIT

FIG. 5

FIG. 6

START

ACQUIRING
EACH DATA — S201

PREDICTING
REACTION SPEED
CONSTANT — S202

PREDICTING
EQUILIBRIUM
CATALYST ACTIVITY — S203

OUTPUTTING REACTION SPEED
CONSTANT AND EQUILIBRIUM
CATALYST ACTIVITY — S204

END

FIG. 7

```
              ┌─────────────┐
              │    START    │
              └─────────────┘
                     │
         ┌─────────────────────┐
         │      ACQUIRING      │  ─── S211
         │     EACH DATA       │
         └─────────────────────┘
                     │
         ┌─────────────────────┐
         │     RELEARNING      │  ─── S212
         │    SECOND MODEL     │
         └─────────────────────┘
                     │
         ┌─────────────────────┐
         │     RELEARNING      │  ─── S213
         │     THIRD MODEL     │
         └─────────────────────┘
                     │
         ┌─────────────────────┐
         │   SRORING MODEL     │  ─── S214
         └─────────────────────┘
                     │
              ┌─────────────┐
              │     END     │
              └─────────────┘
```

FIG. 8

FIG. 9

FIG. 10

20

DECOMPOSED GAS AND
DECOMPOSED NAPHTHA

DECOMPOSED GAS

23

DISTILLATION
COLUMN

DECOMPOSED
LIGHT OIL

DECOMPOSED
HEAVY OIL

RAW OIL

25

REACTOR

COKE

24

HEATING
FURNANCE

FIG. 11

20

RECYCLE HYDROGEN

MAKE-UP HYDROGEN

HEAVY OIL

26

REACTOR

27

HYDROGEN
SEPARATION
TANK

DECOMPOSITION
PRODUCT

MAKE-UP
CATALYST

CATALYST

FIG. 12

2

FIG. 13

INFORMATION PROCESSING DEVICE

*50*

*110*

COMMUNICATION
UNIT

*530*

*131* CONTROL UNIT *536*

*541*

RECEPTION
CONTROL UNIT

GENERATION
UNIT

THIRD
CALCULATION UNIT

*520*

STORAGE UNIT

*521*

FIRST DB

*522*

SECOND DB

*523*

THIRD DB

*132*

TRANSMISSION
CONTROL UNIT

*533*

ACQUISITION
UNIT

*534*

FIRST
CALCULATION UNIT

*535*

ANALYSIS
UNIT

*537*

SECOND
CALCULATION UNIT

*538*

LEARNING
UNIT

*539*

INPUT UNIT

*540*

PREDICTION
UNIT

*542*

OPTIMIZATION
UNIT

*543*

OUTPUT
UNIT

FIG. 14

_521

| FIRST DATA DB | | | | | |
|---|---|---|---|---|---|
| I D | SUBSTANCE | INFORMATION REGARDING SUBSTANCE | OPERATION PARAMETER | CATALYTIC ACTIVITY | . . . |
| 001 | SUBSTANCE A | A1.file | 001.param | 001.cat | . . . |
| | SUBSTANCE B | B1.file | | | . . . |
| | . . . | . . . | | | . . . |
| 002 | SUBSTANCE A | 21.file | 002.param | 002.cat | . . . |
| | SUBSTANCE B | B2.file | | | . . . |
| | . . . | . . . | | | . . . |
| . . . | . . . | . . . | . . . | . . . | . . . |

FIG. 15

522

| SECOND DATA DB | | | |
|---|---|---|---|
| I D | PARAMETER OF FIST REACTION STATE | PARAMETER OF THIRD REACTION STATE | · · · |
| 001 | 011. Param | 111. Param | · · · |
| 002 | 012. Param | -- | · · · |
| 003 | 013. Param | 113. Param | · · · |
| · · · | · · · | · · · | · · · |

FIG. 16

523

**THIRD DATA DB**

| I D | MODEL NAME | PARAMETER | DATE AND TIME | · · · |
|-----|------------|-----------|---------------|-------|
| 001 | MODEL 1 | 201. Param | 20210110 | · · · |
| 002 | MODEL 2 | 202. Param | 20210114 | · · · |
| 003 | MODEL 1 | 203. param | 20210210 | · · · |
| · · · | · · · | · · · | · · · | · · · |

FIG. 17

REACTION SPEED DEPENDENCE

SUBSTANCE
DIFFUSION
DEPENDENCE

CURRENT LOCATION

CATALYST ADSORPTION
DEPENDENCE

FIG. 18

```
        ┌─────────────────┐
        │      START      │
        └────────┬────────┘
                 │
        ┌────────┴────────┐
        │    ACQUIRING    │── S501
        │  LEARNING DATA  │
        └────────┬────────┘
                 │
        ┌────────┴────────┐
        │   CALCULATING   │── S502
        │ PARAMETER OF APPARENT │
        │  REACTION STATE │
        └────────┬────────┘
                 │
        ┌────────┴────────┐
        │  LEARNING MODEL │── S503
        └────────┬────────┘
                 │
        ┌────────┴────────┐
        │  STORING MODEL  │── S504
        └────────┬────────┘
                 │
        ┌────────┴────────┐
        │       END       │
        └─────────────────┘
```

FIG. 19

```
                    ┌─────────────┐
                    │    START    │
                    └──────┬──────┘
                           │
              ┌────────────────────────┐
              │      CALCULATING       │  ─ S601
              │    FIRST REACTION      │
              │    SPEED CONSTANT      │
              └────────────┬───────────┘
                           │
              ┌────────────────────────┐
              │       ANALYZING        │  ─ S602
              │    REACTION SPEED      │
              └────────────┬───────────┘
                           │
              ┌────────────────────────┐
              │    DISPLAYING EACH     │  ─ S603
              │   RATE-LIMITING STAGE  │
              └────────────┬───────────┘
                           │
              ┌────────────────────────┐
              │      QUANTIFYING       │  ─ S604
              │   EACH  RATE-LIMITING  │
              │        STAGE           │
              └────────────┬───────────┘
                           │
              ┌────────────────────────┐
              │      GENERATING        │  ─ S605
              │    MODIFIED MODEL      │
              └────────────┬───────────┘
                           │
              ┌────────────────────────┐
              │  CALCULATING THIRD     │  ─ S606
              │   REACTION SPEED       │
              │      CONSTANT          │
              └────────────┬───────────┘
                           │
                    ┌─────────────┐
                    │   RETURN    │
                    └─────────────┘
```

FIG. 20

START

INPUTTING INFORMATION OF SUBSTANCE AND OPERATION PARAMETER — S701

ACQUIRING LEARNED MODEL — S702

PREDICTING SECOND REACTION SPEED CONSTANT — S703

CALCULATING PRODUCT YIELD — S704

CALCULATING OPERATING PARAMETER — S705

OUTPUTTING OPERATING PARAMETER — S706

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002329187 A
- US 2006047347 A1

- WO 2021157667 A1